# EUROPEAN PATENT SPECIFICATION

(11) **EP 0 815 245 B1**
(45) Date of publication and mention of the grant of the patent: **02.04.2008**
(21) Application number: 96905439.4
(22) Date of filing: 07.02.1996
(51) Int. Cl.: C12N 15/62, C07K 14/54, C07K 19/00, A61K 48/00, C12N 15/24, C12N 15/83, A61K 38/20

(54) **BIOACTIVE IL-12 FUSION PROTEINS**
BIOAKTIVE IL-12 FUSIONSPROTEINE
PROTEINES DE FUSION IL-12 BIOACTIVES

(30) Priority: 08.02.1995 US 385335
(43) Date of publication of application: 07.01.1998
(73) Proprietor: WHITEHEAD INSTITUTE FOR BIOMEDICAL RESEARCH, Cambridge, MA 02142 (US)
(72) Inventor: LIESCHKE, Graham, J., Cambridge, MA 02139 (US); MULLIGAN, Richard, C., Lincoln, MA 01773 (US)
(74) Representative: Arends, William Gerrit
(86) International application number: PCT/US1996/001787
(87) International publication number: WO 1996/024676

(56) References cited:
- EP-A- 0 433 827
- EP-A- 0 614 982
- WO-A-94/13806
- EUROPEAN JOURNAL OF IMMUNOLOGY , vol. 25, no. 1, January 1995, pages 137-146, XP000574005 A. MARTINOTTI ET AL: "CD4 T cells inhibit in vivo the CD8-mediated immune response against murine colon carcinoma cells transduced with Interleukin-12 genes"
- PROCEEDINGS OF THE NATIONAL ACADEMY OF SCIENCES OF USA, vol. 85, August 1988, WASHINGTON US, pages 5879-5883, XP002006553 J.S. HUSTON ET AL: "Protein engineering of Antibody binding sites : recovery of specific activity in anti-digoxin single-chain Fv analogue produced in Escherichia coli" cited in the application
- PROCEEDINGS OF THE NATIONAL ACADEMY OF SCIENCES OF USA, vol. 88, May 1991, WASHINGTON US, pages 4143-4147, XP002006554 U. GUBLER ET AL: "Coexpression of two distinct genes is required to generate secreted bioactive cytotoxic lymphocyte maturation factor"

## Description

This application relates to bioactive fusion proteins and to their utility in tumour therapy.

Production of therapeutic proteins, such as those which are dimeric, is often difficult, inefficient and expensive. Production of a dimer may require separate expression of the two components, followed by joining of those components to form a functional dimer. Alternative methods of producing functional dimeric proteins would be useful.

Eur. J. Immunol. (1995) 25:137-146 describes the expression of the two sub-units of interleukin-12 genes (p35 and p40) from a plasmid wherein p35 and p40 are fused by an internal ribosomal entry site, such that p35 and p40 are not linked by amino acids after expression.

J. Bio. Chem. Vol. 268, 31, 23455-23459, 1993 discloses the construction of a cDNA encoding a "single-subunit" Na pump in which the α and β subunits of the pump were joined by a linker of 17 amino acids.

There are disclosed herein fusion proteins which comprise at least two polypeptide monomers (chains of amino acids) joined through a polypeptide linker and are bioactive, as well as to their production. In some parts of the disclosure, the bioactive fusion proteins comprise two or more polypeptides which occur as subunits or monomers in a corresponding bioactive native dimeric protein and are linked through heterologous amino acid residues (amino acid residues which are not present between two subunits in the native protein). As it occurs in nature, the cytokine IL-12 is a heterodimer made up of a 40 kDa subunit (p40) linked by a disulfide bond to a 35 kDa subunit (p35). Gillessen. S. et al. , Eur. J Immunology, 25:200-206 (1995.) ; Ozmen et al., J. Exp. Med, 180: 907--915 (1995); Heinsel et al., Inf. & Immun., 62(10): 4244-4249 (1994). For example, the fusion protein is a bioactive interleukin-12 (IL-12) fusion protein which comprises two subunits, designated p35 and p40, joined by a polypeptide linker. Also disclosed is a fusion protein which comprises the subunits of other dimeric hematopoietic growth factors joined by a polypeptide linker, or the subunits of other dimeric cytokine proteins joined by a polypeptide linker, Also disclosed is a bioactive fusion protein which comprises two subunits which are bioactive monomers (e.g., interleukin-2, GMCSF) in their native form and are joined through a polypeptide linker to produce a fusion protein which is chimeric or hybrid in nature in that it comprises at least two components or subunits which do not occur together in a native protein (e.g., an interleukin-2/GMCSF fusion protein).

There are also disclosed methods of producing , such fusion proteins, constructs useful in their production and host cells containing the constructs from which the encoded fusion proteins are expressed. The fusion proteins are expressed in an appropriate expression system, such as by a retrovirus vector which contains and expresses DNA encoding the subunits or monomers and the polypeptide linker of the desired fusion protein in an appropriate host cell, such as in mammalian cells. There are also disclosed cells which have been transduced to secrete IL-12 fusion proteins of the present invention, and particularly tumor cells which have been transduced to secrete an IL-12 fusion protein. Also disclosed is the use of the transduced tumor cells, particularly in the treatment of tumors.

Our fusion proteins are useful for the same purposes (e.g., therapeutic or diagnostic uses) as the corresponding native protein. For example; IL-12 fusion proteins can be used to enhance the lytic activity of NK/lymphokine - activated killer cells, act as a growth factor for activated human T and NK cells and stimulate production of IFN-γ by resting peripheral blood mononuclear cells (PBMC). IL-12 is also useful in treating a variety of cancers. For instance, IL-12 is useful for the enhancement of antitumor immunity and, as described herein, tumor cells which secrete either native IL-12 or an IL-12 fusion protein of the present invention can be used to treat established tumors, such as to prevent the further development of a tumor, cause established tumors to regress, prolong survival, or a combination thereof. The fusion proteins have certain advantages over the corresponding native proteins in that they can be made efficiently and reproducibly by the methods described herein. Furthermore, our fusion proteins may also have advantages over the corresponding native proteins in terms of modified or enhanced activity, more favourable bioavailability and improved pharmacokinetic properties.

According to a first aspect, the invention provides DNA comprising DNA encoding IL-12 p35 subunit, preferably of human or mouse origin, encoding a polypeptide linker and DNA encoding IL-12 p40 subunit, preferably of human or mouse origin, wherein the DNA encoding the polypeptide linker is positioned between the DNA encoding the IL-12 p35 subunit and the DNA encoding the IL-12 p40 subunit and wherein expression of the DNA results in production of a bioactive IL-12 fusion protein comprising the IL-12 p35 subunit and the IL-12 p40 subunit joined by the encoded polypeptide linker.

In a second aspect of the invention, we provide a bioactive IL-12 protein which comprises IL-12 p35 subunit, preferably of human or mouse origin, and IL-12 p40 subunit, preferably of human or mouse origin, joined by a polypeptide linker, preferably comprising 7 to 16 amino acid residues.

There is provided, according to a third aspect of this invention, a method of producing a bioactive IL-12 protein comprising the steps of:
a) providing an expression vector comprising DNA encoding IL-12 p35 subunit, preferably of human or mouse origin, DNA encoding a polypeptide linker, preferably of 7 to 16 amino acid residues, and DNA encoding IL-12 p40 subunit, preferably of human or mouse origin, wherein the DNA encoding the polypeptide linker is positioned between the DNA encoding the IL-12 p35 subunit and the DNA encoding the IL-12 p40 subunit;
b) introducing the expression vector into an appropriate host cell;
c) maintaining the host cell resulting from step b) under conditions appropriate for expression of the DNA present in the expression vector, resulting in production of a bioactive IL-12 protein in which the two subunits are joined by the polypeptide linker.

There are disclosed herein IL-12-secreting tumour cells, preferably selected from CMS-5 tumour cells and B16 tumour cells, for use in therapy to treat a subject having a disorder characterised by an established tumour, preferably by reducing the size of the tumour, more preferably by 50% or more, prolonging survival of the subject compared with an untreated subject, or both.

There are also disclosed tumour cells secreting a bioactive IL-12 protein which comprises IL-12 p35 subunit and IL-12 p40 subunit joined by a polypeptide linker, for use in therapy to treat a subject having a disorder characterised by an established tumour, or to prevent the establishment of a tumour in a subject by administration to said subject after the initiation of the tumour and before the establishment of the tumour.

There is also disclosed the manufacture of a medicament for treating an established tumour, a fibrosarcoma, a melanoma or a renal cell carcinoma, of IL-12-secreting tumour cells, preferably CMS-5 cells, B16 cells or renal cell carcinoma cells.

In the accompanying drawings:

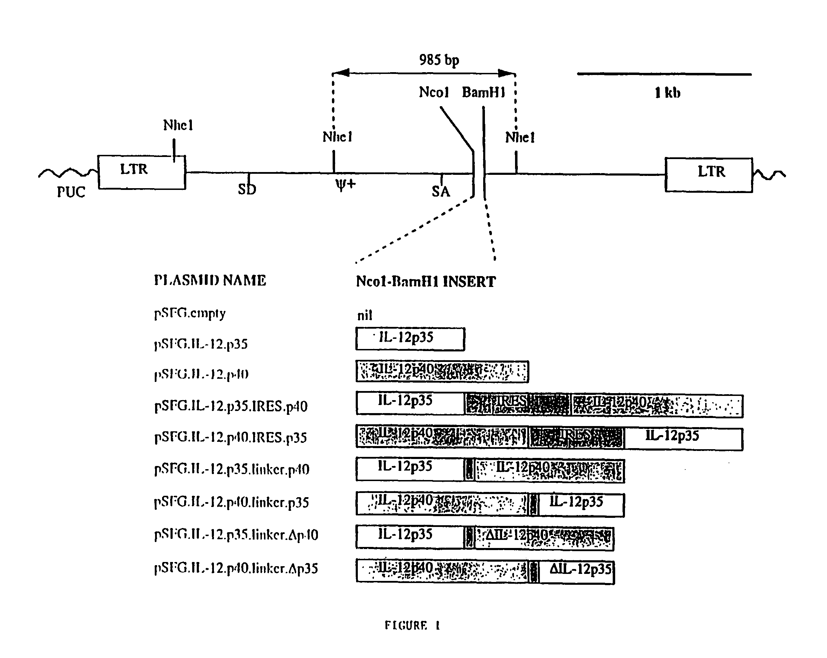

Figure 1 shows the structures of SFG-based retroviral constructs for interleukin-12 production (SD=splice donor; IRES=internal ribosome entry site; SA=splice acceptor; LTR=long terminal repeat).

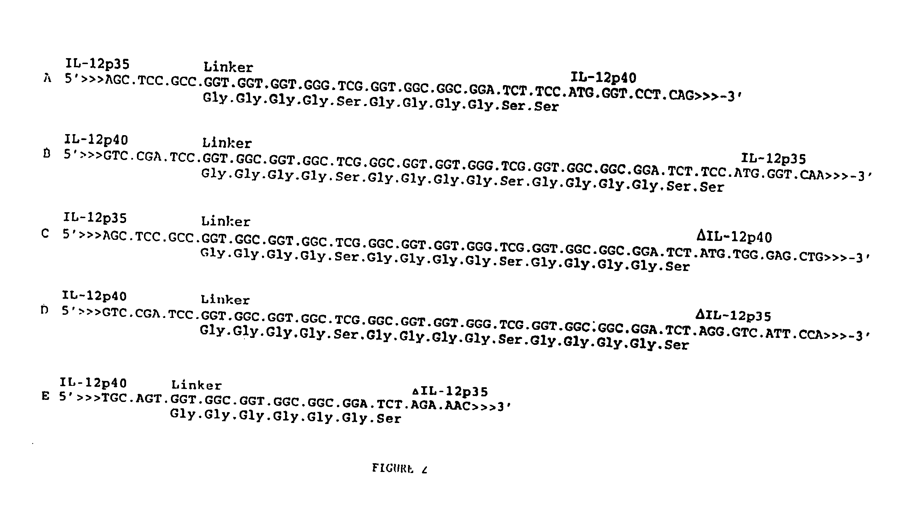

Figure 2 shows the nucleic acid sequences encoding the linker sequences in interleukin-12 fusion proteins of the present invention and flanking IL-12 p35 and IL-12 p40 sequences (SEQ ID NO: 1 to 4 and 35), as well as the encoded amino acid sequences (SEQ ID NO: 5 to 7 and 36).

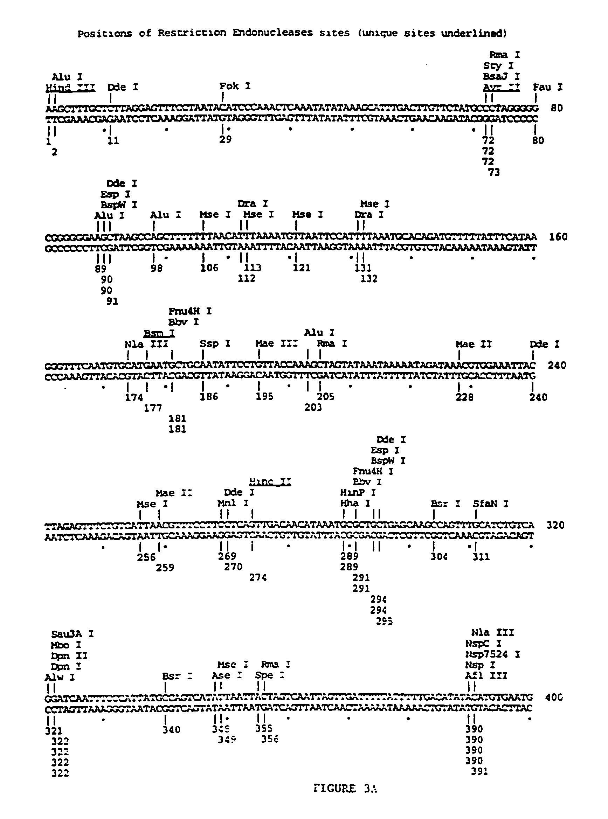

Figures 3A-3U show the full restriction map and the nucleic acid sequence (SEQ ID NO: 8 and 9) of pUC19-SFG.

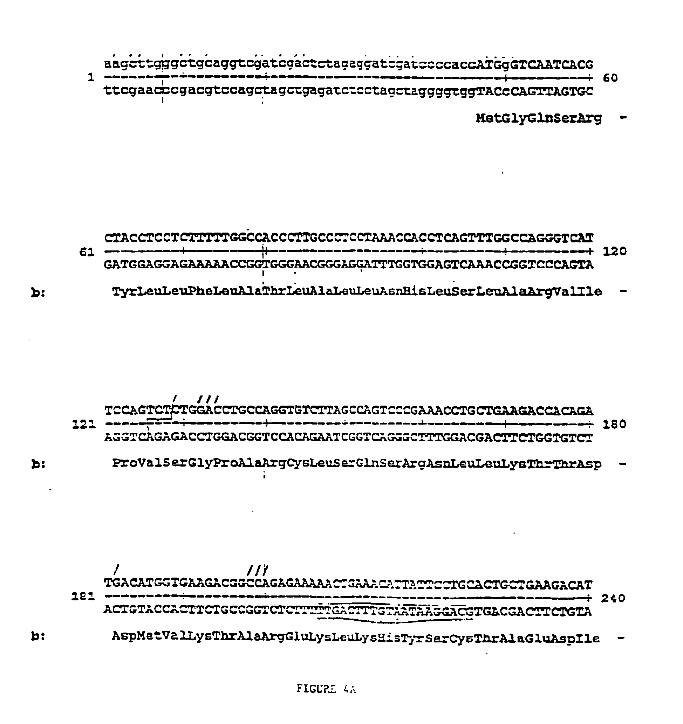

Figures 4A-4C show the nucleic acid sequence (SEQ ID NO: 10 and 11) encoding the murine IL-12 p35 subunit and the amino acid sequence of the murine IL-12 p35 subunit (SEQ ID NO: 12).

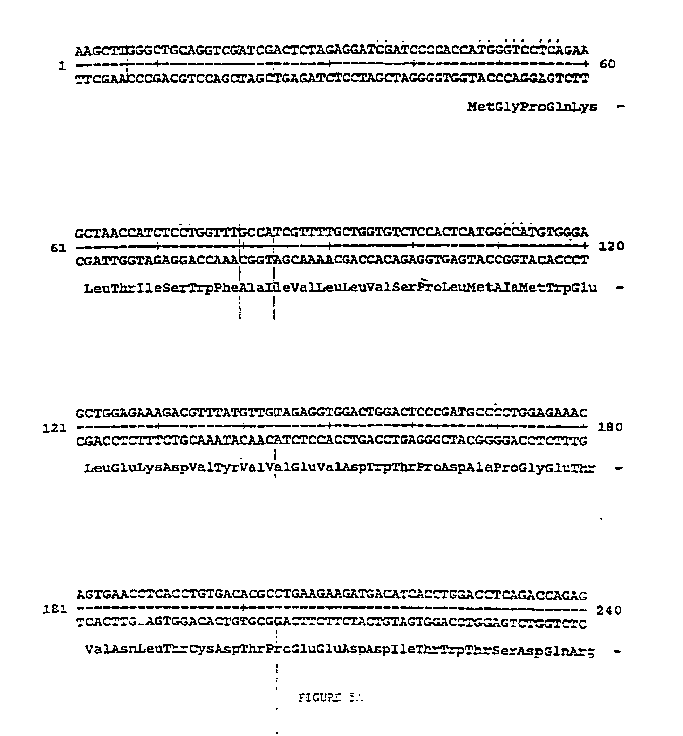

Figures 5A-5D show the nucleic acid sequence (SEQ ID NO: 13 and 14) encoding murine IL-12 p40 subunit and the amino acid sequence (SEQ ID NO: 15) of the murine IL-12 p40 subunit.

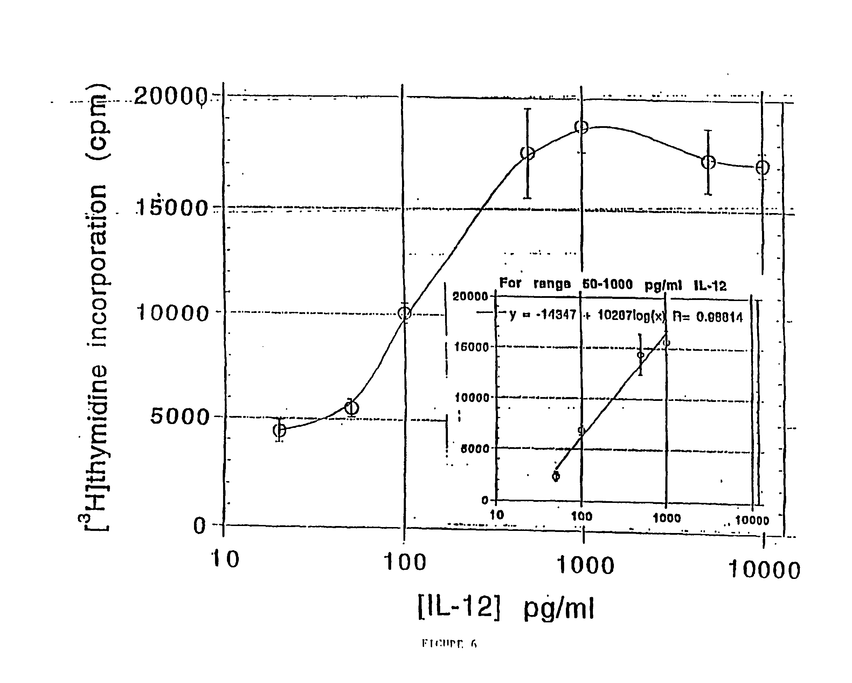

Figure 6 shows a standard curve generated using recombinant murine IL-12.

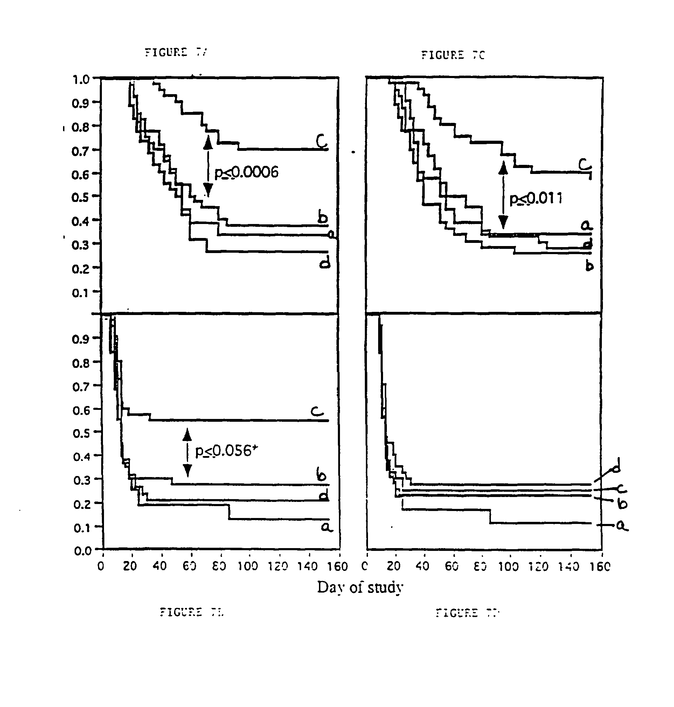

Figures 7A-7D show graphic representations of the effect of immunotherapy of CMS-5 tumor-bearing mice with wild-type, GM-CSF- and IL-12-secreting CMS-5 cells. Treatment was started either on day 7 (7A and 7B) or day 14 (7C and 7D) after tumor challenge. Endpoints are either survival (7A and 7C) or tumor-free survival (7B and 7D). Tumors were either untreated (a) or treated with GM-CSF-secreting CMS-5 cells (b), IL-12-secreting CMS-5 cells (c) or wild type CMS-5 cells (d).

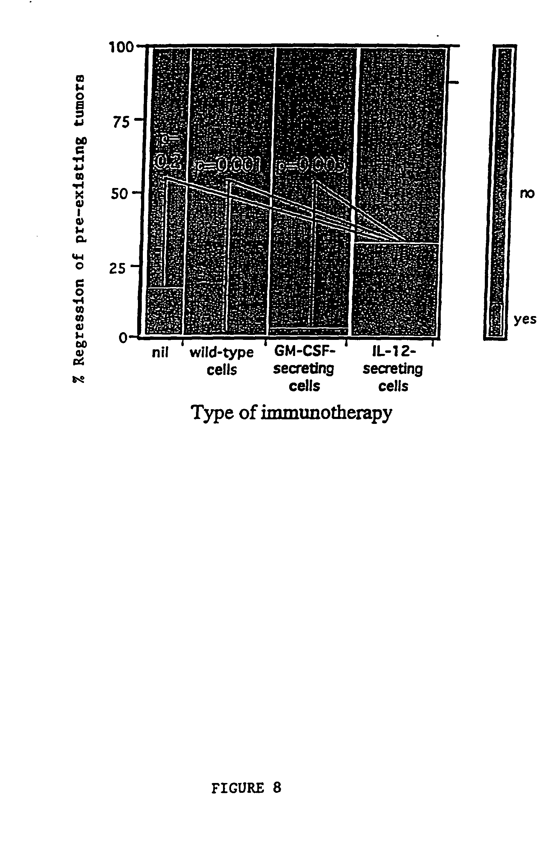

Figure 8 is a graphic representation of the incidence of regression of established CMS-5 tumors by type of immunotherapy. Tumors were treated as follows: column 1 received no immunotherapy; column 2 was treated with wild-type tumor cells; column 3 was treated with GM-CSF-secreting tumor cells; and column 4 was treated with IL-12-secreting tumor cells.

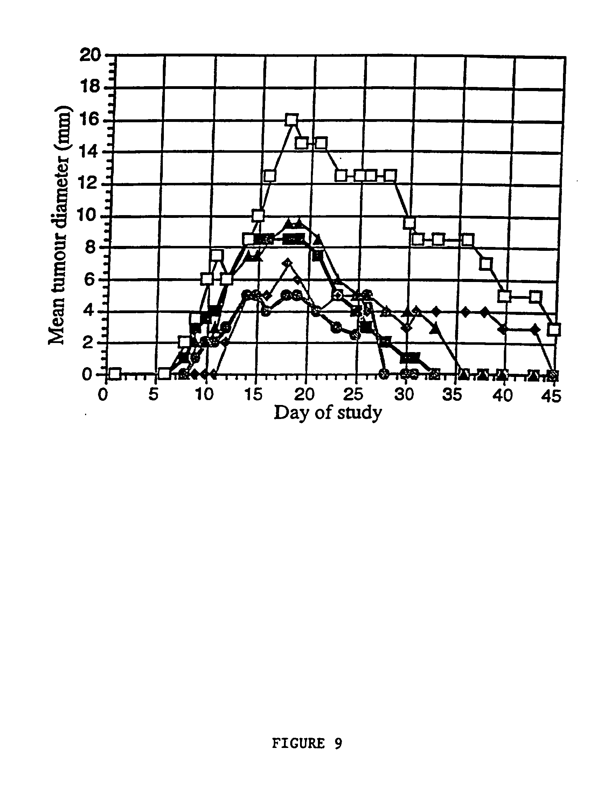

Figure 9 is a graphic representation of tumor regression in mice treated with systemic IL-12. The open square and closed square, triangle, circle and diamond represent 5 individual mice treated with systemic IL-12 at 0.1 µg/d given 5 days per week for 4 weeks.

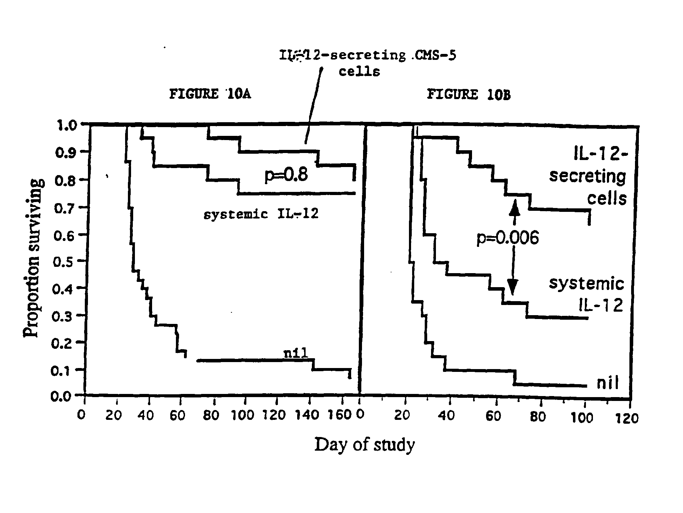

Figures 10A-10B show graphic representations of the superior survival resulting from immunotherapy with IL-12-secreting CMS-5 cells compared to systemic IL-12 administration or no treatment (nil). Figure 10A depicts results using a tumor inoculum of 2 x 10⁵ cells. Figure 10B depicts results using a tumor inoculum of 4 x 10⁵ cells.

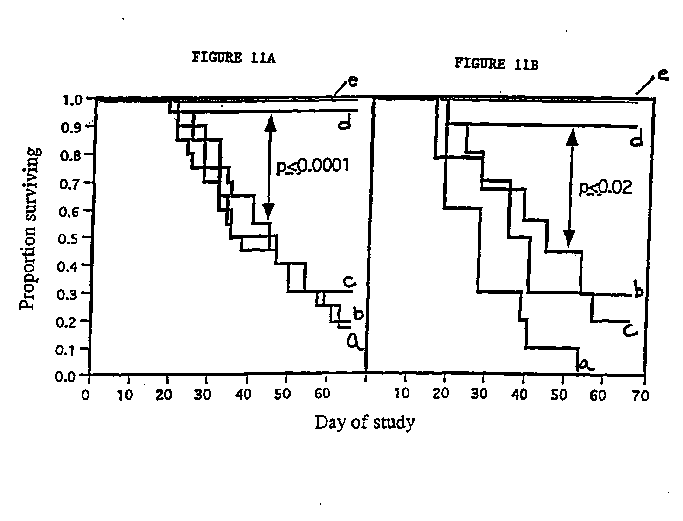

Figures 11A-11B show graphic representations of the comparison of efficacy (proportion of mice surviving) of CMS-5 cells secreting different forms of IL-12 as immunotherapy for established CMS-5 tumors. Figure 11A shows results using tumors initiated by 2 x 10⁵ CMS-5 cells with treatment starting on day 14 (20 mice per group pooled from two experiments). Figure 11B shows results using tumors initiated by 4 x 10⁵ CMS-5 cells with treatment starting on day 14 (1 group of 10 mice). The tumors were either untreated (a) or treated with wild type CMS-5 cells (b), GM-CSF-secreting CMS-5 cells (c), native IL-12-secreting CMS-5 cells (d) or IL-12 fusion protein-secreting CMS-5 cells (e).

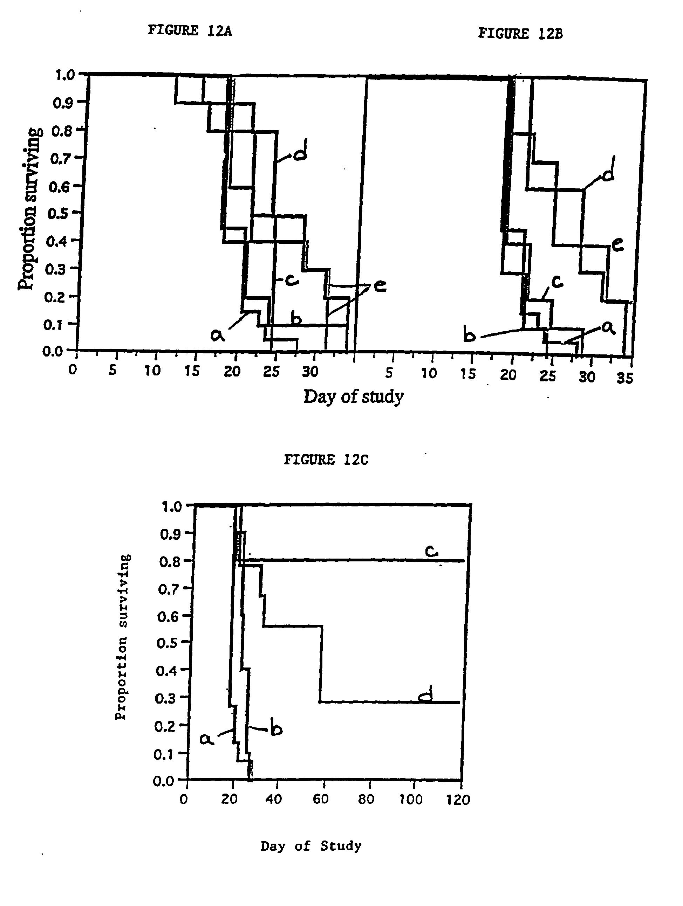

Figures 12A-12C are graphic illustrations of the results of immunotherapy of B16 (melanoma) tumors with cytokine-secreting tumor cells. For the pre-existing tumor model, tumors were initiated with 4 x 10⁵ B16 cells and immunotherapy commenced on day 7 (Figure 12A) or day 14 (Figure 12B). For the challenge model (Figure 12C), 5 x 10⁵ irradiated cells were administered as a vaccine 14 days before tumor challenge with 1 x 10⁶ B16 cells. Tumors were either untreated (a) or treated with wild type B16 cells (b), GM-CSF-secreting B16 cells (c), native IL-12-secreting B16 cells (d) or IL-12 fusion protein-secreting B16 cells (e) .

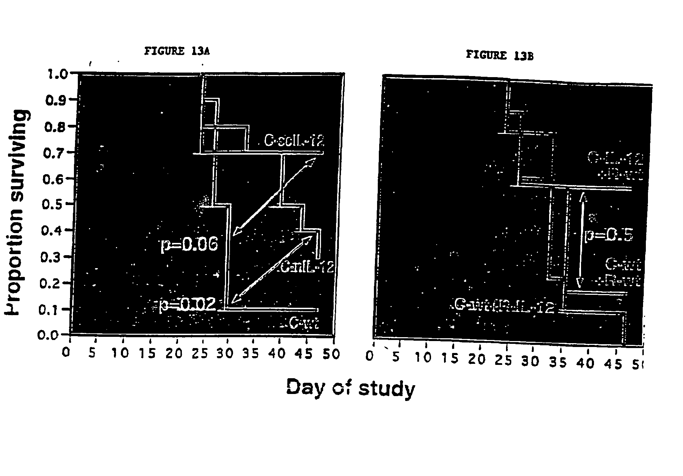

Figures 13A-13B are graphic illustrations of the effects on immunotherapy of IL-12 delivery by different cell types in mice with pre-existing renal cell carcinoma (RENCA) tumors. Figure 13A shows results when RENCA tumors were treated with either irradiated wild-type CMS-5 tumor cells (C-wt) or CMS-5 tumor cells transduced to secret either native IL-12 (C-nIL-12) or the IL-12 fusion protein (C-scIL-12). Figure 13B shows results when RENCA tumors were treated with either a combination of wild-type CMS-5 and RENCA cells (C-wt + R-wt), a combination of IL-12 fusion protein-secreting RENCA cells and wild type CMS-5 cells (C-wt + R-IL-12) or a combination of IL-12 fusion protein-secreting CMS-5 cells and wild type RENCA cells (C-IL-12 + R-wt).

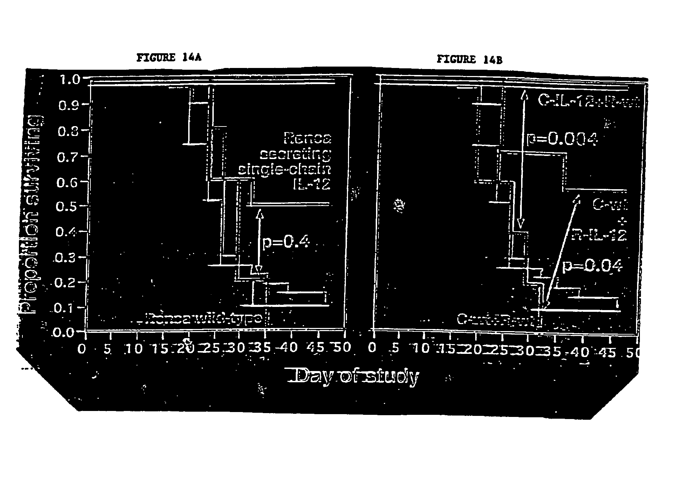

Figures 14A-14B are a graphic representation of the effects on immunotherapy of pre-existing CMS-5 tumors with IL-12 fusion protein-secreting RENCA tumor cells. Figure 14A shows the results when CMS-5 tumors were treated with either wild type RENCA cells or RENCA cells transduced to secrete the IL-12 fusion protein. Figure 14B shows the results when CMS-5 tumors were treated with either a combination of wild type RENCA and wild type CMS-5 cells (C-wt + R-wt), a combination of IL-12 fusion protein-secreting RENCA cells and wild type CMS-5 cells (C-wt + R-IL-12) or a combination of IL-12 fusion protein-secreting CMS-5 cells and wild type RENCA cells (C-IL-12 + R-wt).

Described herein are bioactive fusion proteins which comprise at least two subunits linked or joined by an intervening amino acid linker, a method of producing the bioactive fusion proteins, constructs useful for producing the fusion proteins which can be expressed in host cells, and host cells containing the constructs.

In one disclosure, the bioactive fusion proteins of comprise: 1) at least two polypeptide subunits or monomers which correspond to polypeptide subunits present in a native dimeric protein which has a specified bioactivity and 2) at least one polypeptide linker which joins the subunits in such a manner that the resulting fusion protein is bioactive. If the resulting fusion protein is dimeric (includes two subunits or monomers), the two components can be-subunits which occur in the same native dimeric protein (e.g., two IL-12 subunits); subunits which occur in two different native dimeric proteins (e.g., one subunit from IL-12 and one subunit from IL-3) or monomers which are individually bioactive (e.g., IL-2, GMCSF). Multimeric fusion proteins, which comprise three or more subunits joined by polypeptide linkers, can comprise, for example, three or more of the subunits which occur in the same native dimeric protein (e.g., three or more IL-12 subunits), three or more subunits which occur in different native dimeric proteins (e.g., two IL-12 subunits and one IL-3 subunit), three or more bioactive monomers (e.g., three IL-2 monomers, two IL-2 monomers and one GMCSF monomer) or a combination of subunits from native dimeric proteins and bioactive monomers (e.g., two IL-12 subunits and a GMCSF monomer). In each case, a polypeptide linker is present between two subunits (e.g., the order is subunit-linker-subunit-linker-subunit). As used herein, the terms subunit and monomer are used interchangeably to refer to the components of a dimeric or multimeric protein and the single component of a monomeric protein. The order of subunits in the fusion protein of the present invention can be p35-linker-p40 or p40-linker-p35. In either case, the polypeptide linker is positioned between the two subunits. A bioactive fusion protein of the present invention which includes subunits which occur in the same native dimeric protein "mimics" or is similar to what is referred to herein as a corresponding native dimeric protein in terms of its bioactivity, but differs from the corresponding native dimeric protein in that the fusion protein includes linker amino acid residues which do not occur in the corresponding native protein (heterologous amino acid residues) between each pair of polypeptide subunits. A corresponding native protein is one which includes the subunits present in the fusion protein and exhibits biological activity also exhibited by the fusion protein.

In the bioactive IL-12 fusion protein of the present invention, the two subunits, designated p35 and p40, of a mammalian native IL-12 protein (e.g., human, mouse, rat, dog, cat, monkey, chimpanzee or pig IL-12 protein) are joined through a polypeptide linker. Here, the corresponding native protein is the mammalian native IL-12 protein. Similarly, in the case of another bioactive fusion protein, such as IL-3, the corresponding native protein is IL-3. The amino acid residues of the subunits of the bioactive fusion protein can be the same as those of the subunits of the corresponding native protein or can be different, provided that the resulting fusion protein exhibits the desired bioactivity. For example, the subunit(s) can have a different amino acid sequence from that of the corresponding subunit of a native protein (i.e., the sequence of the native subunit can differ in that one or more amino acid residues has been deleted or replaced by a naturally-occurring or non-naturally-occurring amino acid residue, additional amino acid residues have been incorporated, or an amino acid residue has been modified). The desired bioactivity is activity like that of the corresponding native protein (e.g., it produces a physiological response which also results from the activity of the corresponding native protein). The bioactivity of a fusion protein (e.g., the duration of its effect, extent of the resulting response) may be greater or lesser than that of the corresponding native protein.

The polypeptide linker present in the fusion protein can be of any length and composition appropriate to join two subunits in such a manner that the resulting fusion protein has the desired biological activity and retains its integrity as a dimer or multimer The appropriate length and composition of a linker can be determined empirically for the specific fusion protein to be produced. Generally, the polypeptide linker will be at least 7 amino acid residues, although it can be shorter (e.g., 2 to 6 amino acid residues). Typically the linker will be less than 30 amino acid residues in length, such as 7 to 25 amino acid residues or 7 to 20 amino acid residues in length. In one embodiment, the polypeptide linker is 7 to 16 amino acid residues and in specific embodiments is 7, 11, 15 or 16 amino acid residues. Specific linkers used in producing bioactive IL-12 fusion proteins are represented in Figure 2 and described in Example 4. In specific embodiments, the polypeptide linkers are exemplified by the sequences (Gly₄Ser)₃; (Gly₄Ser)₃Ser; (Gly₄Ser)₂Ser and Gly₆Ser, and these linkers can be used to join subunits of IL-12 fusion proteins of the present invention. Alternatively, other polypeptide linkers can be used to join two IL-12 subunits to produce a bioactive IL-12 fusion protein.

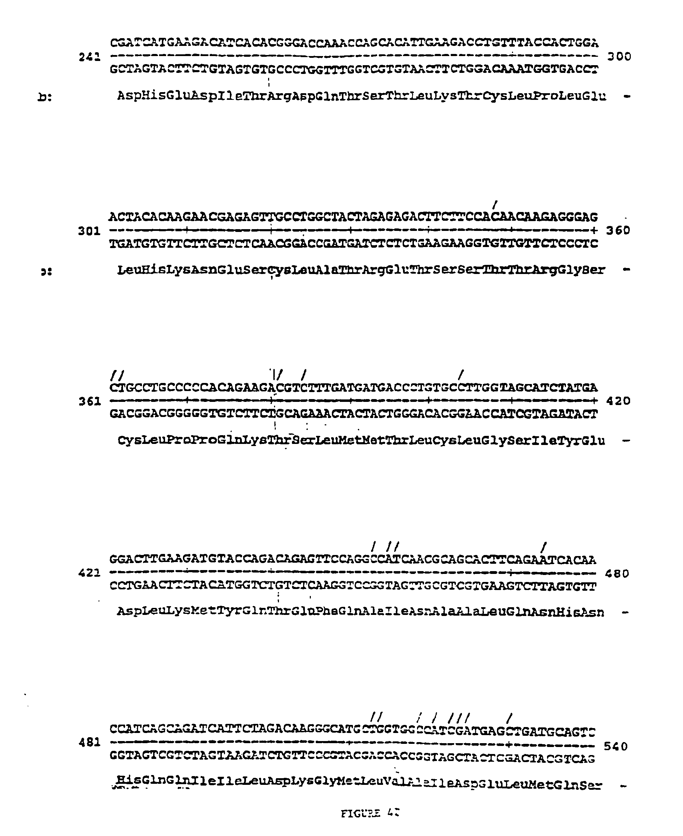

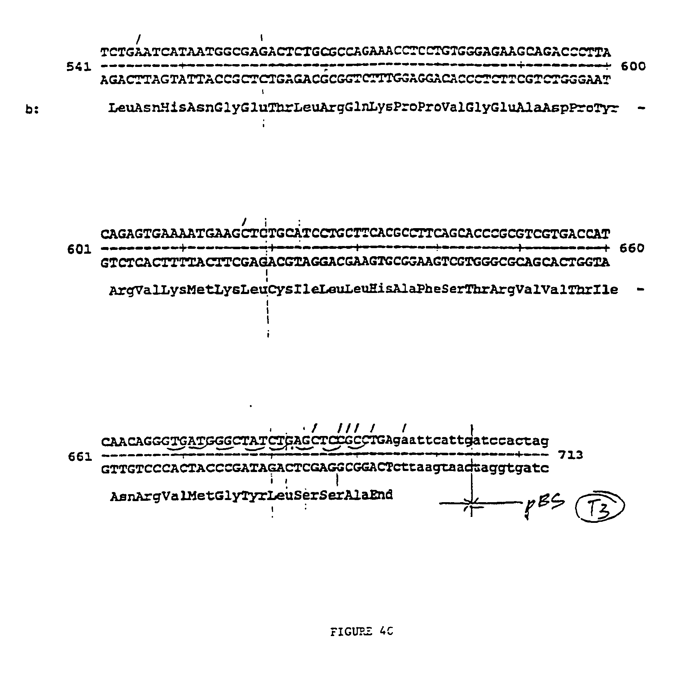

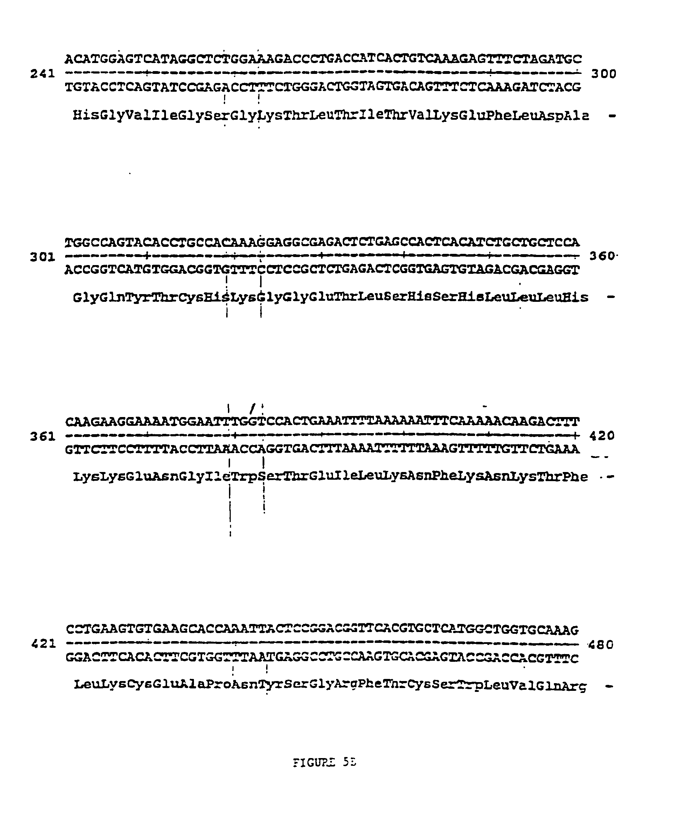

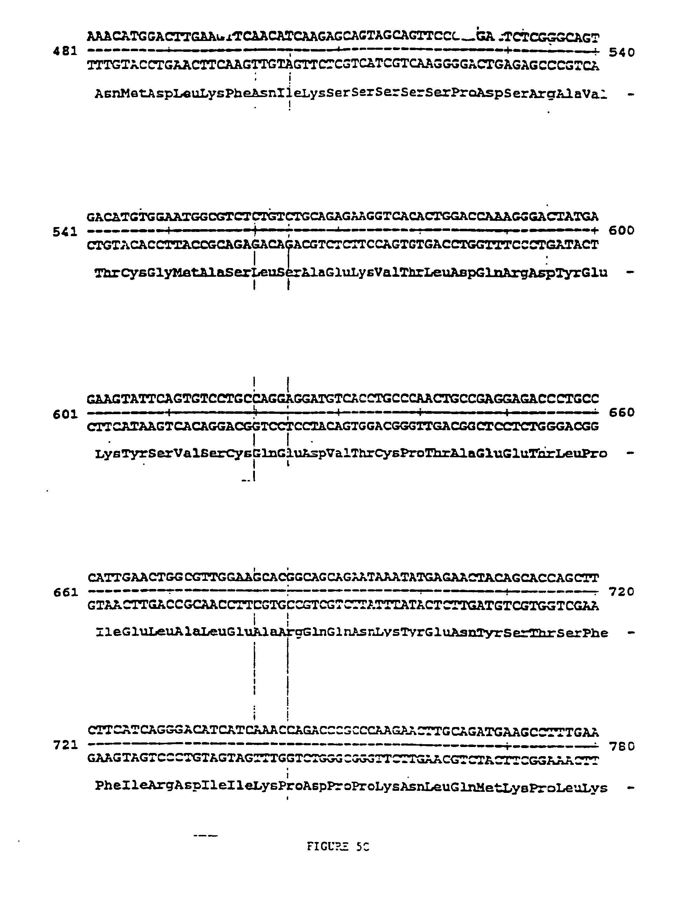

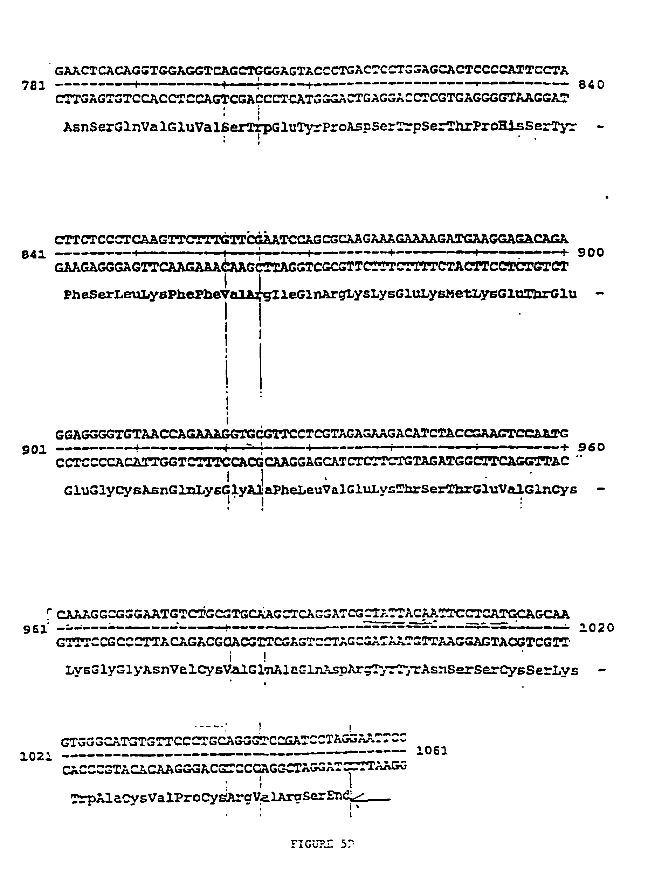

The DNA encoding the bioactive fusion protein can be cDNA or genomic DNA and can be from a variety of animals, particularly mammals. For example, the DNA can be human, mouse, rat, dog, cat, monkey, chimpanzee, pig or ferret DNA. The DNA can encode a complete or entire subunit (e.g., a complete IL-12 p35 subunit and a complete IL-12 p40 subunit) or a fragment or portion of a subunit(s), provided that the encoded fusion protein has the desired biological activity when it is expressed. The nucleic acid sequences of DNA encoding mouse IL-12 p35 and p40 subunits are represented in Figures 4 and 5, respectively. The nucleic acid sequences of DNA encoding human IL-12 p35 and p40 subunits have been published (Gubler et al. in Proceedings of the National Academy of Sciences USA, 88:4143 (1991); Figure 4A-4C and SA-5D). All or a portion of IL-12 DNA can be used to produce the subject IL-12 fusion protein, provided that the encoded fusion protein is bioactive (has IL-12 activity).

Any expression system appropriate for expressing a protein such as a mammalian, bacterial, yeast or insect expression system, can be used to express the fusion proteins of the present invention. For example, as described herein, a viral (e.g., a retroviral) vector which expresses DNA (e.g., cDNA) encoding the desired fusion protein in a mammalian host cell has been used. As also described herein, retroviruses containing cDNA encoding the p35 and p40 subunits of IL-12 and an intervening polypeptide linker (an IL-12 fusion protein) have been constructed and transfected into packaging cells (e.g., BOSC23 packaging cells). Target cells (e.g., CMS-5 fibrosarcoma cell line) were infected with virus-containing supernatants and cultured; media conditioned by infected cells was assayed for IL-12 activity using an interleukin-2 and concanavalin-A primed splenocyte proliferation bioassay. Packaging or producer cell lines other than BOSC23 cells can be used to produce infectious retroviruses containing the fusion protein-encoding DNA. In addition, target cells other than a fibrosarcoma cell line, such as B16 melanoma or renal cell carcinoma cell lines, can be used to produce the fusion protein. IL-12 bioactivity was demonstrable in cells infected with the retroviruses, as described in Example 4.

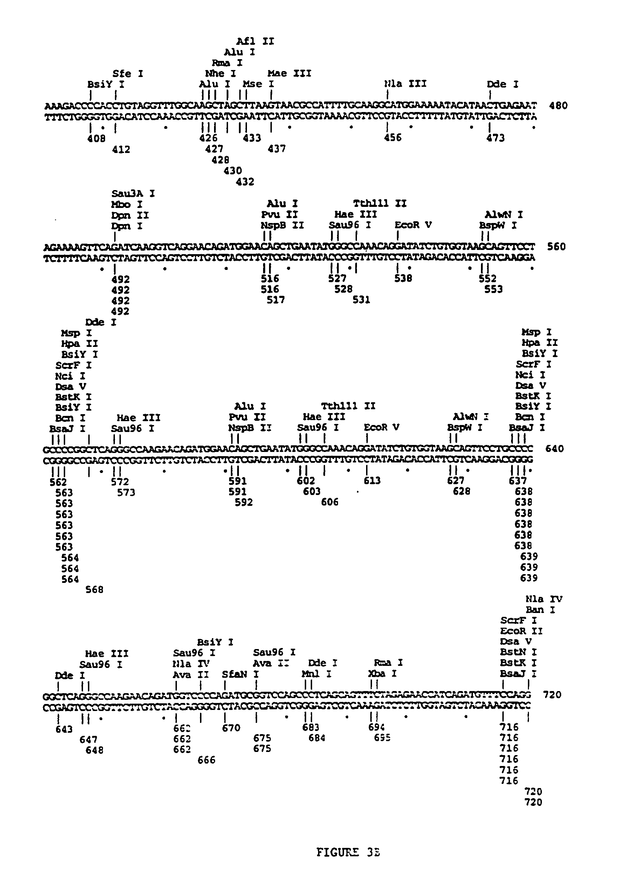

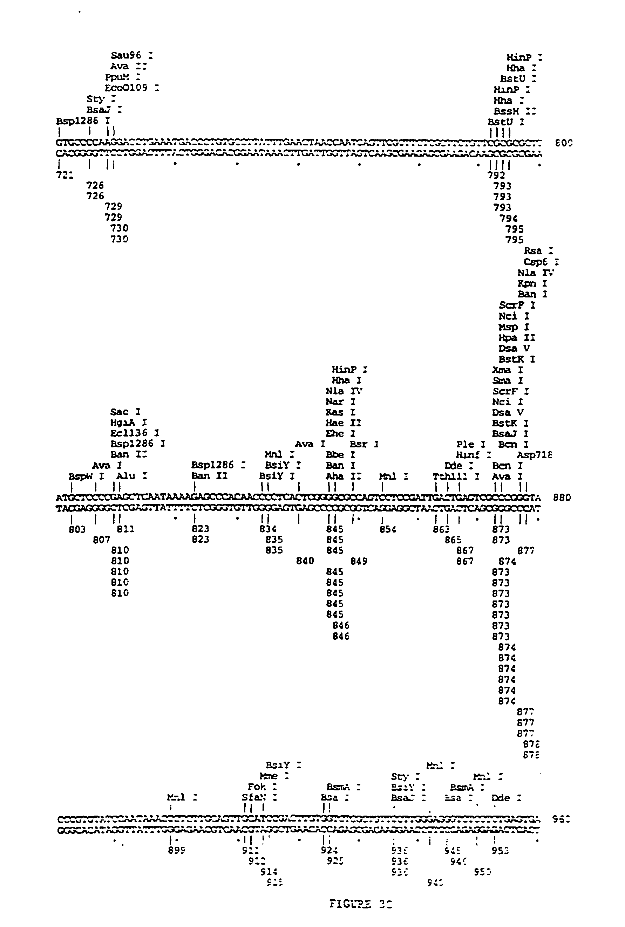

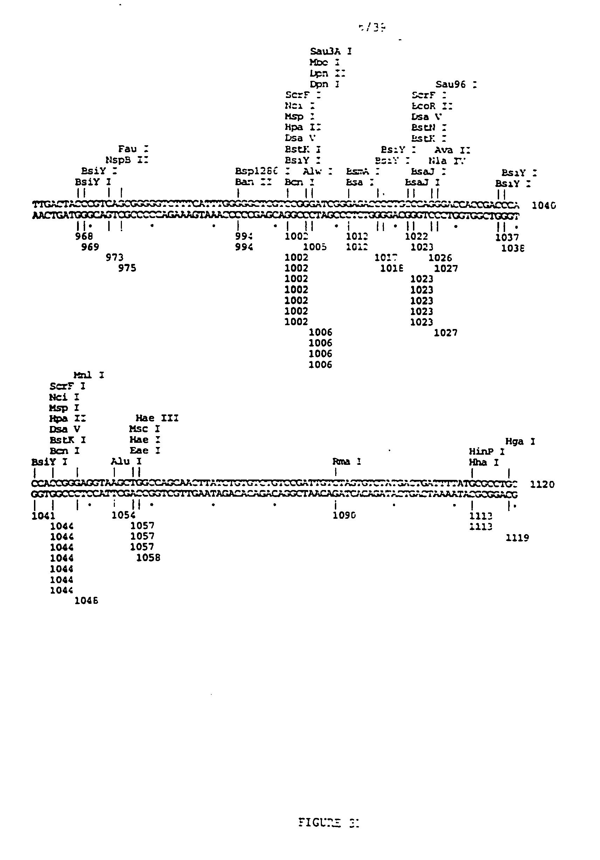

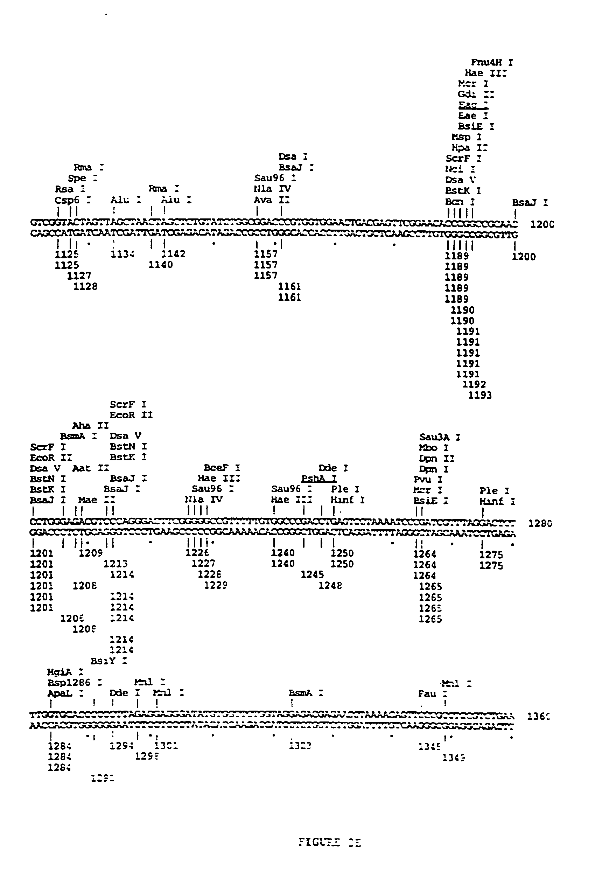

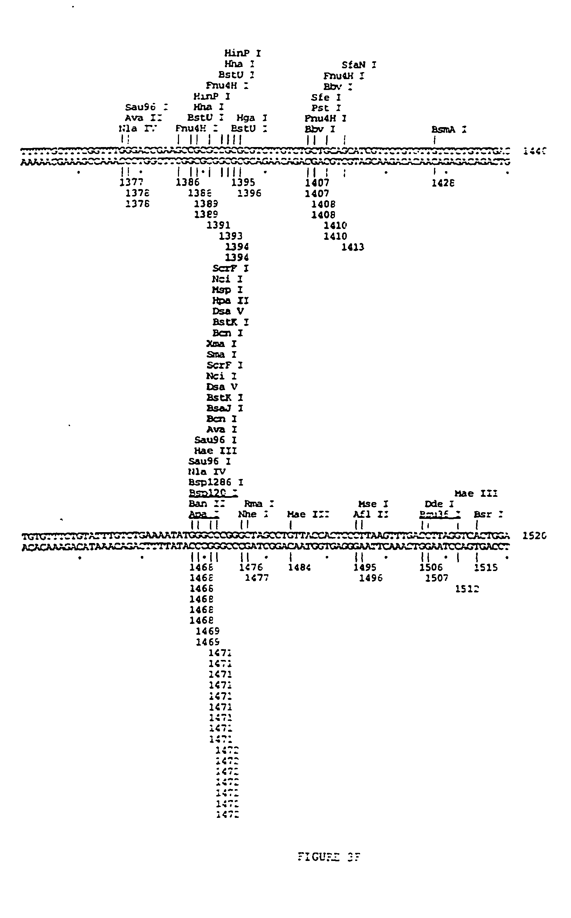

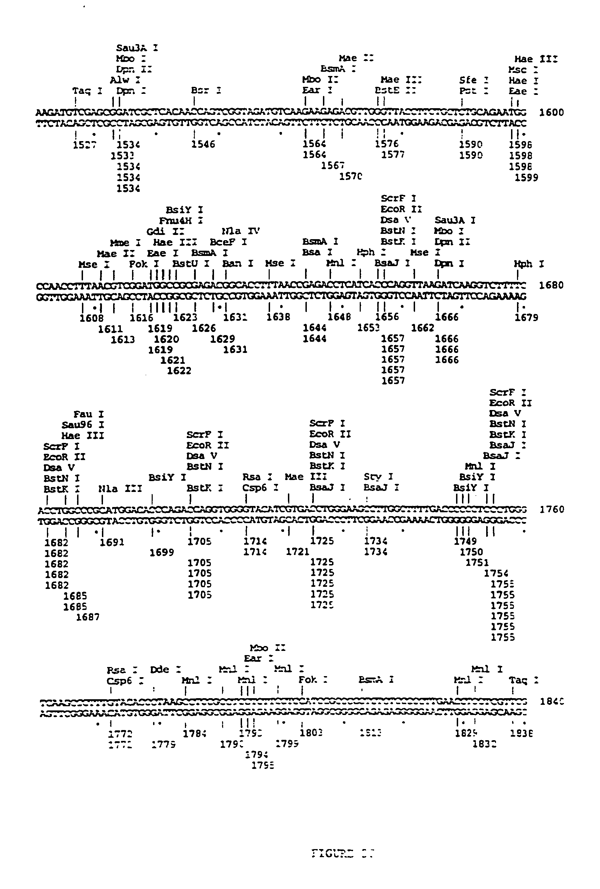

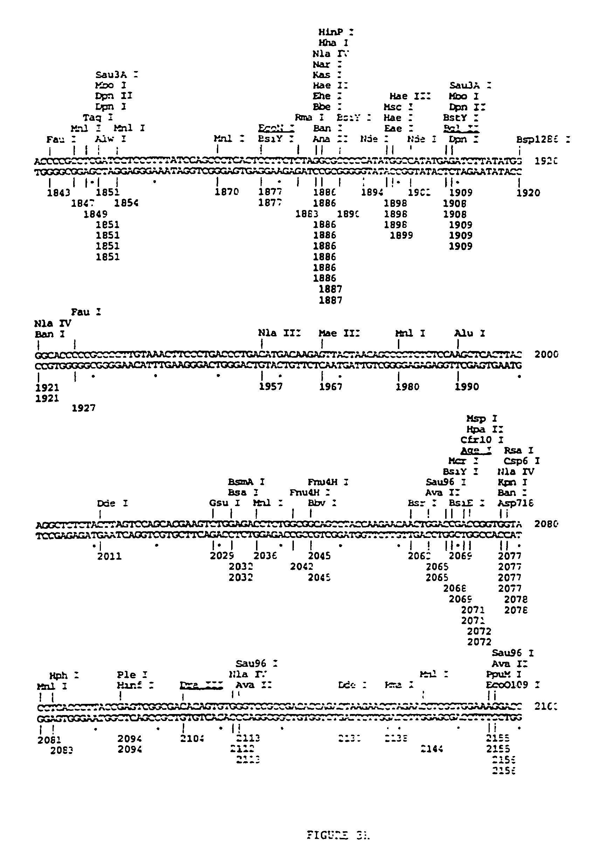

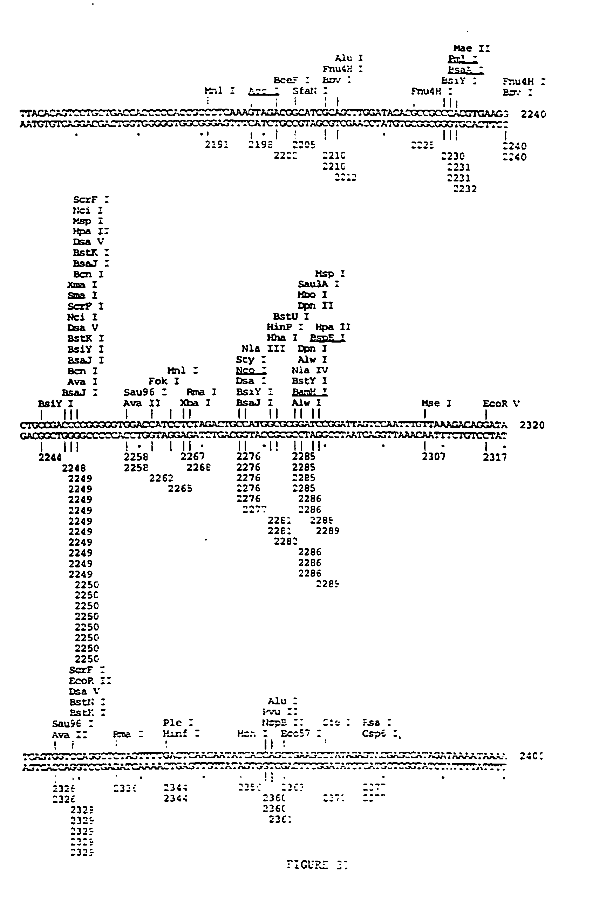

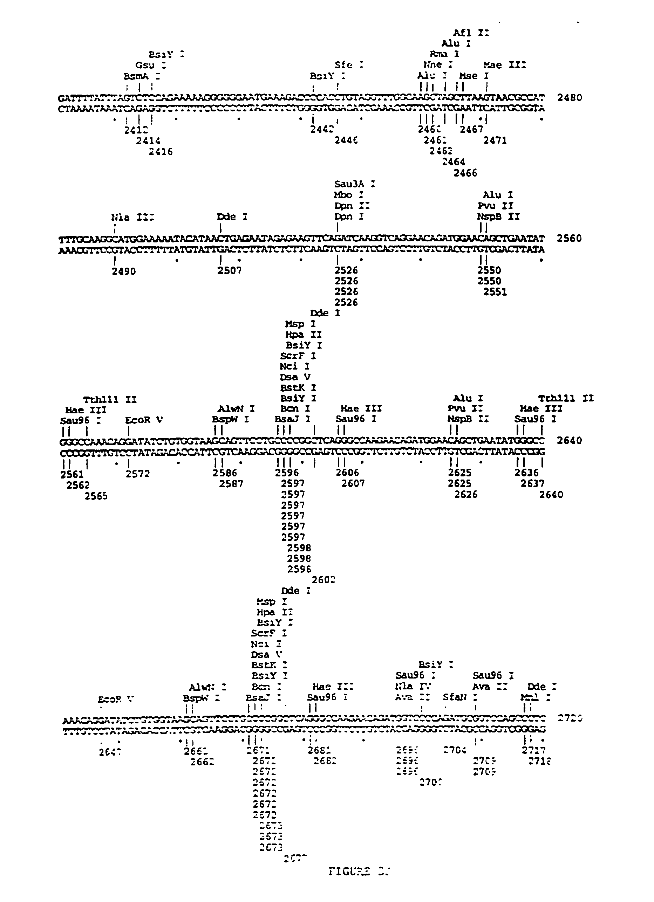

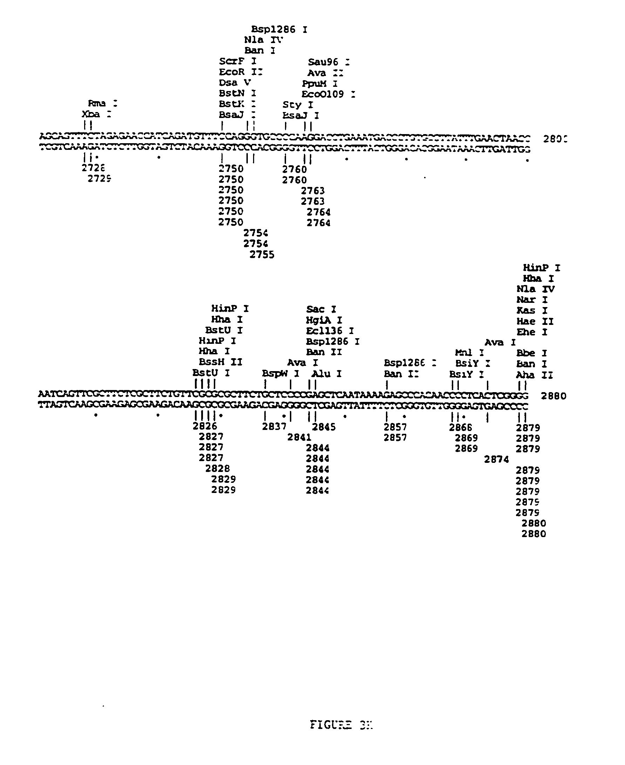

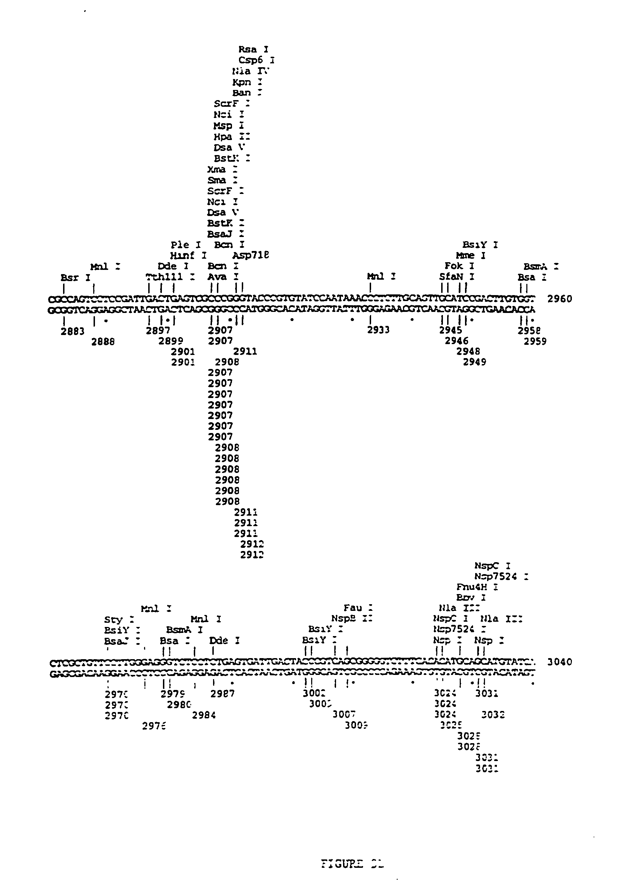

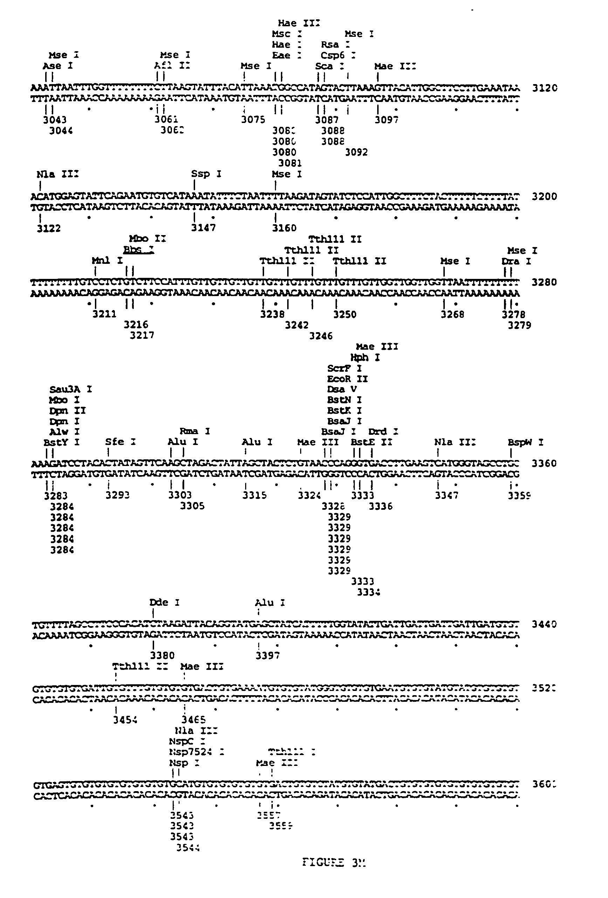

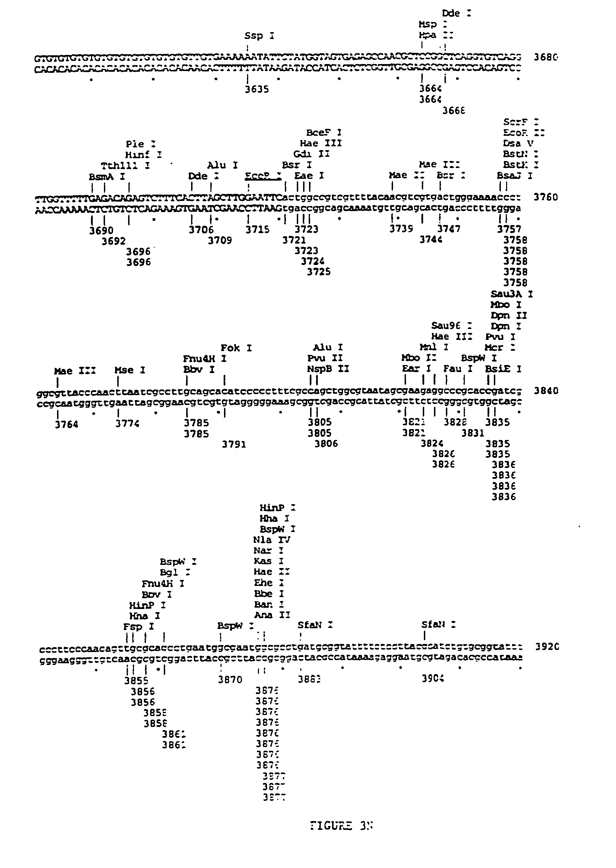

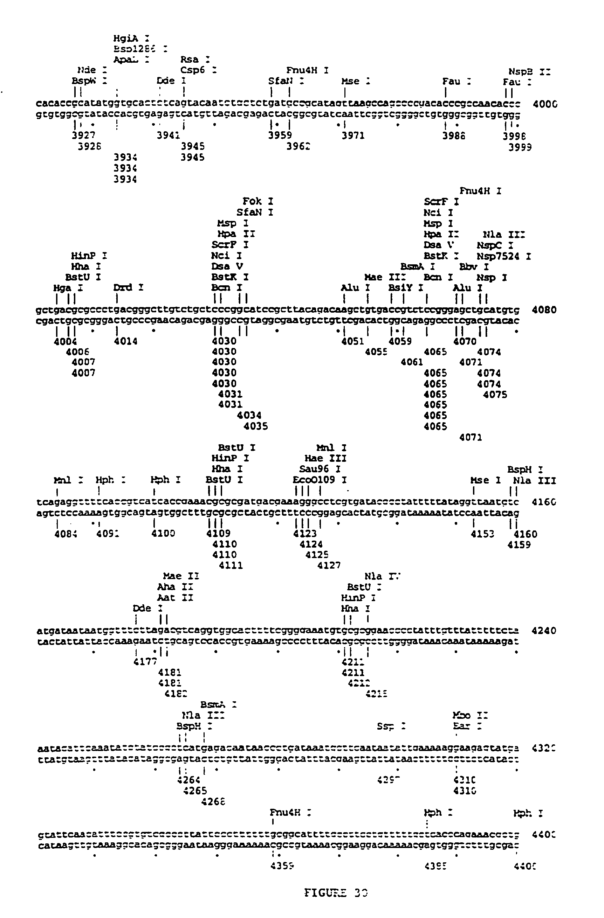

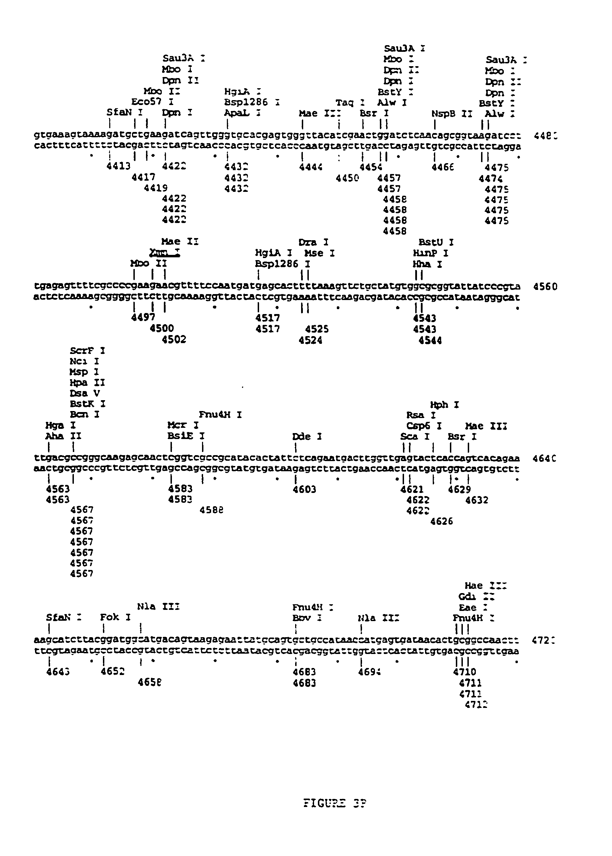

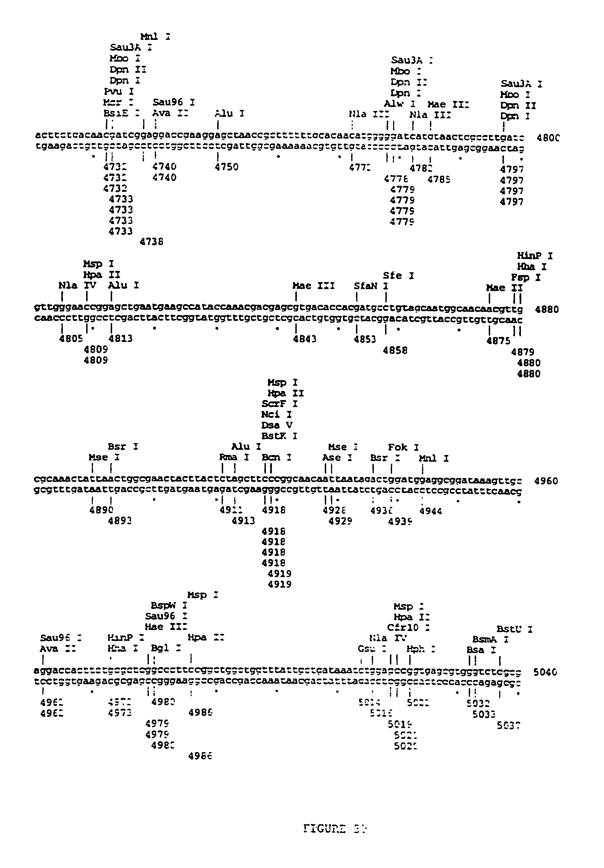

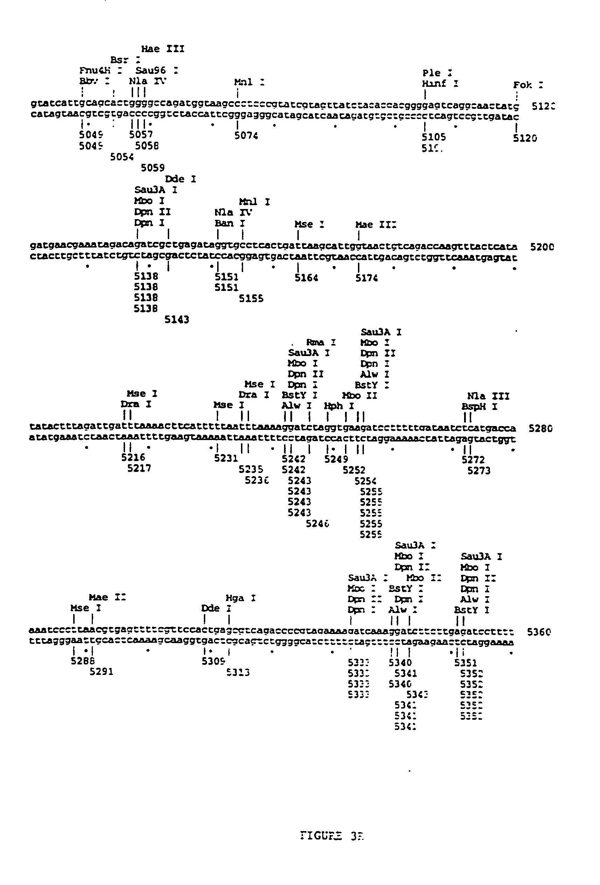

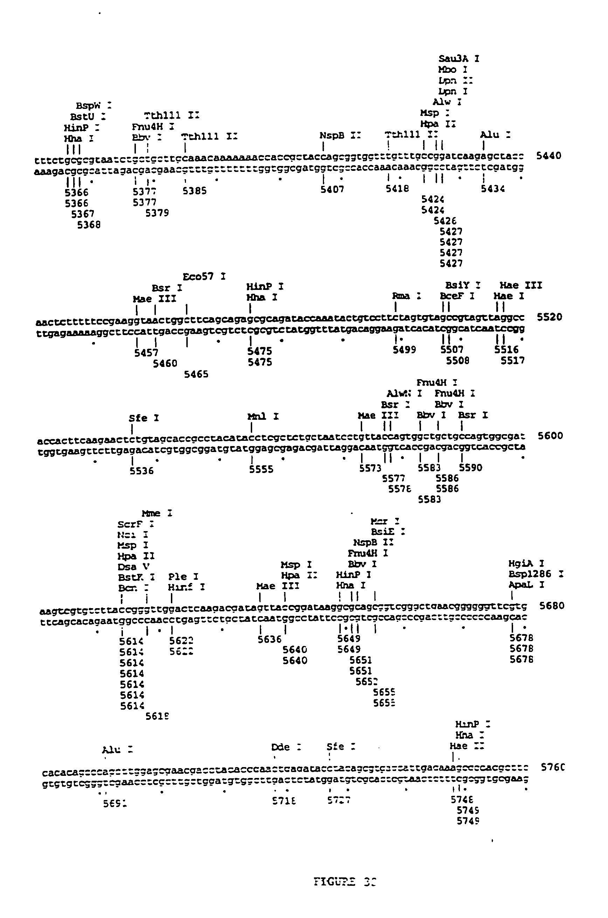

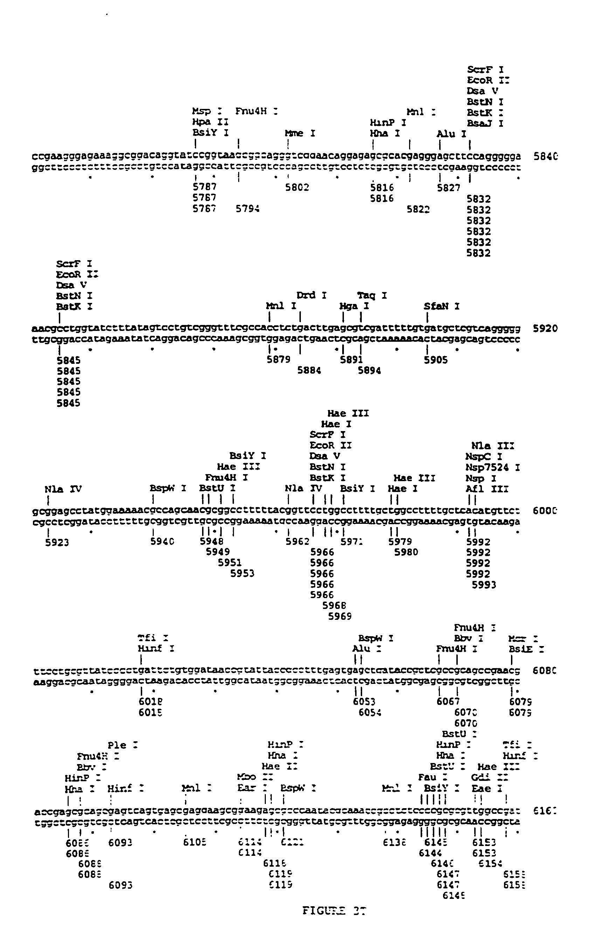

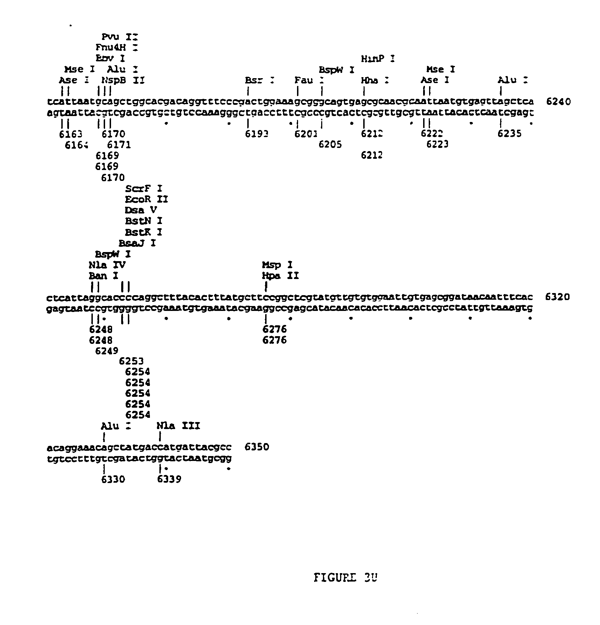

Specific retroviruses have been constructed for expression of an IL-12 fusion protein (Example 1 and Figure 1) and cells infected with the retroviruses have been shown to produce bioactive IL-12 fusion proteins (see Example 4). The retroviruses used all included the SFG retroviral backbone whose sequence is shown in Figure 3. The vectors designated pSFG.IL-12.p35 and pSFG. IL-12.p40 include, respectively, the cDNA for the IL-12 p35 subunit or the cDNA for the IL-12 p40 subunit. The vector designated pSFG.IL-12p35-IRES-p40 includes cDNA encoding the IL-12 p35 subunit and cDNA encoding the IL-12 p40 subunit, separated by an internal ribosome entry site sequence. The vector designated pSFG.IL-12p40-IRES-p35 includes the same components as plasmid pSFG.IL-12p35-IRES-p40 but the dimers are in the reverse order, as indicated. The vectors designated pSFG.IL-12.p35-linker-p40 and pSFG.IL-12.p40-linker-p35 include cDNAS encoding each IL-12 subunit linked by the (Gly₄Ser)₂Ser and (Gly₄Ser) ₃Ser linker respectively. The vectors designated pSFG.IL-12.p35-linker-Δp40 and pSFG.IL-12.p40-linker-Δp35 include linked cDNAs in which sequences encoding a putative 22 amino acid leader sequence were deleted from the second cDNA. The vector designated pSFG.hIL-12.p40.1inker.Δp35 is a human form of the IL-12 fusion protein and is analogous to the murine form pSFG.IL-12.p40.linker.Δp35 except that the linker is shorter due to a deletion which occurred during the cloning (see Figure 2, construct E). As described in Example 4, IL-12 bioactivity was shown in conditioned medium from cells infected with the retroviruses.

Prokaryotic and eukaryotic host cells transfected by the described vectors are also disclosed. For instance, cells which can be transfected with the vectors of the present invention include, but are not limited to , bacterial cells such as *E*. *coli,* insect cells (baculovirus), yeast or mammalian cells such as Chinese hamster ovary cells (CHO). Tumor cells which are transduced to secrete the IL-12 fusion proteins of the present invention are particularly useful.

Thus, expression vectors described herein can be used to transform, transfect or transduce host cells, either eukaryotic (yeast, avian, insect or mammalian) or prokaryotic (bacterial cells), using standard procedures used in producing other well known proteins. Similar procedures, or modifications thereof, can be employed to prepare recombinant proteins according to the present invention by microbial means or tissue-culture technology. For example, fibroblast-derived 3T3 cells can be transduced with the vectors of the present invention to express and secrete the IL-12 fusion proteins of the present invention. Tumor cells and 3T3 cells are useful in the context of the present invention, as cells transduced to secrete IL-12 or IL-12 fusion proteins of the present invention are a useful source of the protein or fusion protein (e.g., for purification). Tumor cells transduced to secrete IL-12 or IL-12 fusion proteins also have particular utility as antitumor agents as described herein.

The tumor cells to be transduced can be selected from the individual to be treated or from another individual; furthermore, the tumor cells to be transduced can be the same type as the tumor cells of the tumor to be treated or the tumor cells can be of a different type from the tumor to be treated. For example, a CMS-5 tumor can be treated with CMS-5 tumor cells, renal cell carcinoma (RENCA) tumor cells, or B16 tumor cells which secrete IL-12 or an IL-12 fusion protein of the present invention. Alternatively, the tumor can be treated with a combination of IL-12-secreting, IL-12 fusion protein-secreting and wild type cells of the same or different cell type. For example, a RENCA tumor can be treated with a combination of wild type RENCA cells and IL-12 fusion protein-secreting CMS-5 cells or with a combination of native IL-12-secreting CMS-5 cells and IL-12 fusion protein-secreting RENCA tumor cells.

Disclosed herein are transduced tumor cells which express native IL-12 or IL-12 fusion proteins of the present invention and their use in treating tumors. That is, transduced tumor cells which express and secrete IL-12 or IL-12 fusion proteins are useful as therapeutic agents for the treatment of cancer or to treat established tumors, and provide a means for reversing tumors (reducing their size or causing their complete regression) or preventing further growth of an established tumor. As described herein, transduced tumor cells expressing IL-12 or IL-12 fusion proteins of the present invention cause the regression of established tumors, prevent the establishment of tumors, prolong survival or a combination thereof in animals to which they are administered in a therapeutically appropriate dose.

For instance, the tumor cells secreting IL-12 or the IL-12 fusion protein of the present invention can be formulated with a physiologically acceptable medium to prepare a pharmaceutical composition. The particular physiological medium may include, but is not limited to, water, buffered saline, polyols (e.g., glycerol, propylene glycol, liquid polyethylene glycol) and dextrose solutions. The optimum concentration of the active ingredient(s) in the chosen medium can be determined empirically, according to procedures well known to medicinal chemists, and will depend on the ultimate pharmaceutical formulation desired. Methods of introduction of the IL-12-secreting or IL-12 fusion protein-secreting tumor cells include, but are not limited to, intradermal, intramuscular, intraperitoneal, intravenous, subcutaneous, oral and intranasal. The tumor cells secreting native IL-12 or an IL-12 fusion protein can be administered at or near the site of a tumor to be treated or at any other site on the body, provided that the IL-12 or IL-12 fusion protein produces the desired therapeutic effect (regression of established tumors, prevention of tumor establishment or prolonged survival). As described herein, proximity of tumor site and administration site is not necessary for the efficacy of the treatment. Other suitable methods of introduction can also include rechargeable or biodegradable devices and slow release polymeric devices. The pharmaceutical compositions described herein can also be administered as part of a combinatorial therapy with other agents. Treatment regimens will depend upon the dose, route of delivery, frequency with which the composition is administered, type, size and stage of the tumor to be treated, and the age, health and other physical characteristics of the individual to be treated.

For example, a therapeutically effective quantity (dose) of the transduced tumor cells (optionally formulated with a physiologically appropriate medium) is administered to an individual having a tumor to be treated (e.g., decreased in size or prevented from increasing in size). The transduced tumors cells can also be administered in a therapeutically appropriate dose to an individual to prevent the establishment of a tumor. For example, the transduced tumor cells can be administered to an individual in need of anticancer therapy (e.g., an individual with an established tumor or an individual in whom establishment of a tumor is to be prevented). Alternatively, the IL-12 fusion protein of the present invention can be administered directly to the individual in a therapeutically effective dose; the IL-12 fusion protein can be optionally combined with a physiologically acceptable medium as described above.

As described herein, the efficacy of IL-12-secreting tumor cells as antitumor immunotherapy was assessed in mice with established tumor burdens. The immunogenic CMS-5 (fibrosarcoma) and non-immunogenic B16 (melanoma) tumors were used; RENCA tumors were also utilized as described herein.

As shown in Examples 6-8, work described herein demonstrates that for the immunotherapeutic treatment of. 14-day established palpable CMS-5 tumors, immunotherapy with IL-12-secreting and IL-12 fusion protein-secreting tumor cells prolonged survival by inducing the regression of tumors. Furthermore, immunotherapy with IL-12-secreting tumor cells induced tumor regression even when the palpable tumor burden averaged more than 5% of body mass. Although IL-12 has antitumor activity against CMS-5 tumors when administered systemically, for mice with larger tumor burdens at the onset of therapy there was a significant survival advantage of IL-12-secreting tumor cell immunotherapy over systemic IL-12 therapy. This shows that there is an advantage in delivering the IL-12 by transduced tumor cells rather than just as systemic cytokine therapy. Data from tumor cells transduced to express an IL-12 fusion protein (SFG. IL-12. p40. linker. Δp35) indicate that the murine and human forms of the fusion protein have a specific activity at least equal to the native molecule in an in vitro bioassay.

Results described herein show that IL-12-secreting B16 cell vaccination altered the natural history of the growth of later established B16 tumors, and they also appear to be able to enhance immunological mechanisms capable of modulating tumor growth. IL-12-secreting B16 cells are useful as immunotherapy for established B16 tumors, as they effectively prolong survival. These results show that there exist inducible innate mechanisms able to modulate the natural history of established tumors in the mouse, and that IL-12-secreting cells are more potent at inducing them than GM-CSF-secreting cells. These results, which are more fully described in the Examples below, show that IL-12-secreting tumor cells have efficacy as immunotherapy for established tumors.

The present invention is illustrated by the following examples, which are not intended to be limiting in any way.

### EXAMPLE 1 Construction of Plasmids

The general structure of the plasmids used in these studies is shown schematically in Figure 1. The confirmed sequences of the linkers in each of the fusion proteins are given in Figure 2.

### Source of plasmids

The plasmids containing cDNAs for the murine IL-12 p35 and p40 subunits (pBS.IL-12.p35 and pBS.IL-12.p40) were provided by Hoffmann-La Roche (Nutley, NJ). The numbering of base pairs in this document corresponds to the maps of the inserts of these two plasmids (Figures 4 and 5). The plasmid containing the SFG retroviral backbone was provided by Dr. Dan Ory (Whitehead Institute, Cambridge, MA) as pSFG-TPA, a pUC plasmid containing the SFG retroviral backbone between the HindIII and EcoR1 sites with a tissue plasminogen activator cDNA between the unique Ncol and BamH1 sites in the SFG retrovirus. A nucleotide sequence map of the SFG retroviral backbone is shown in Figure 3.

### Plasmid pSFG. IL-12. p35

The IL-12p35 cDNA was provided in pBluescript with the sequences surrounding the translational initiation ATG optimized to ACCATGG according to the rules of Kozak. The IL-12p35 cDNA fragment was excised as a Ncol-EcoRl fragment, the EcoRl overhang having been filled using the Klenow fragment of *E*. *coli* DNA polymerase 1. This fragment was ligated using T4 DNA ligase into the Ncol-BamHl sites of pSFG, the BamH1 overhang having been filled using the Klenow fragment of *E*. *coli* DNA polymerase 1. The resulting plasmid is designated pSFG.IL-12.p35.

### Plasmid nSFG.IL-12.p40

The IL-12p40 cDNA was provided in pBluescript. The Ncol-BamHl fragment containing the IL-12p40 cDNA was excised and ligated into the Ncol-BamHl sites of pSFG to make pSFG.IL-12.p40.

### General Strategy for Construction of SFG-based Vectors

The general strategy for constructing the SFG-based retroviral vectors for IL-12 fusion protein expression is as follows: Two oligonucleotides encoding the sense and antisense strand of a (Gly₄Ser)₃ linker fragment and contiguous IL-12 cDNA sequences to be linked (with terminal sequences for the creation of cohesive ligatable overhangs) were synthesized using a "PCR-mate" 391 DNA synthesizer (Applied Biosystems, Foster City, CA). The sequence of the (Gly₄Ser)₃ linker was that of Huston et al. (Proc. Natl. Acad. Sci. USA, 85-:5879-5883(1988)).

For the two fusion proteins using complete IL-12 cDNAs, the oligonucleotides were designed to be cloned into a unique restriction enzyme site at the 3' end of the first cDNA, reconstructing the 3' end of the first cDNA and enabling a Ncol-Ncol fragment encompassing the full cDNA and linker sequence to be cloned into the Ncol site of the SFG plasmid containing the other cDNA.

The cloning strategy was similar for the two fusion proteins with a deletion of 66 bp coding the first 22 amino acids of the second cDNA. Linker oligonucleotides were designed to be cloned into unique restriction enzyme sites that lay 3' of bp 66 of the translated bases of the second cDNA in the fusion protein construct. This enabled a fragment to be excised for cloning that reconstructed the 3' end of the first cDNA joined to the linker and contained the linker joined to codon 23 of the second cDNA.

The sequence of the linker and contiguous cDNA regions in plasmids was determined using a "Sequenase" kit (Amersham, Cleveland, OH).

### Plasmid pSFG.IL-12.p35-linker-p40

The oligonucleotides were: sense, and antisense,

These two oligonucleotides were annealed, phosphorylated using T4 polynucleotide kinase, and ligated into the Sac1 site of pES.IL-12.p35 which had been dephosphorylated using calf intestinal phosphatase. The Ncol-Ncol fragment of the resulting plasmid containing the IL-12p35 cDNA and correctly orientated linker was excised and ligated into the dephosphorylated Ncol site of pSFG.IL-12p40 to create pSFG.IL-12.p35-linker.p40 (the correct orientation of this ligated fragment was demonstrated by a Sac1 digest).

This plasmid was sequenced using the following two primers: 5'-CAGAGTGAAAATGAAGCT-3' (SEQ ID NO: 18) and 5'-GAAGCTCTGCATCCTGCT-3' (SEQ ID NO: 19), corresponding to bp 601-618 and 613-630 of the IL-12p35 cDNA. Sequencing demonstrated that a deletion had occurred during cloning resulting in a loss of 15 bp from the linker sequences, but maintaining an intact reading frame. The sequence of the linker in this plasmid is given in Figure 2.

### Plasmid pSFG.IL-12.p40.linker.p35

The oligonucleotides were: sense, and antisense,

These two oligonucleotides were annealed and ligated into the Sse83871 and BamH1 sites of pBS.IL-12.p40. The Ncol-Ncol fragment of the resulting plasmid containing the IL-12p40 cDNA and correctly orientated linker was excised and ligated into the dephosphorylated Ncol site of pSFG.IL-12p35 to create pSFG.IL-12.p4O.linker.p35 (the correct, orientation of this ligated fragment was demonstrated by a Xcml digest).

This plasmid was sequenced using the following two primers: 5'-CTATTACAATTCCTCATG-3' (SEQ ID NO: 22) and 5'-GAGGGCAAGGGTGGCCAA-3' (SEQ ID NO: 23), corresponding to base pairs 997-1014 of the IL-12 p40 cDNA and base pairs 91-74 of the IL-12 p35 cDNA (an antisense primer). Sequencing confirmed that the sequence of the linker and contiguous IL-12 cDNA sequences were as expected.

Subsequent restriction enzyme mapping of pSFG.IL-12.p40.linker.p35 after the transfection and expression studies were completed revealed that it probably contained a concatamer of Ncol-Ncol-fragments from the final cloning step.

### Plasmid pSFG.IL-12.p35.linker.Δp40

The oligonucleotides were: sense, and antisense,

These two oligonucleotides were annealed, phosphorylated using T4 polynucleotide kinase, and ligated into pBS.IL-12.p40 from which the 30 base pair 5' Xcm1-Xcm1 fragment had been excised. The Sacl-Sacl fragment from the resultant plasmid was excised and ligated into the Sac1 site of pBS.IL-12.p35 which had been dephosphorylated using calf intestinal phosphatase (the correct orientation of the ligated fragment was demonstrated by a Nco1-EcoR1 digest). The Nco1-EcoR1 fragment of the resultant vector was excised, the EcoR1 overhang having been filled using the Klenow fragment of E. *coli* DNA polymerase 1, and ligated into the Ncol and Klenow-filled BamH1 sites of pSFG to create pSFG.IL-12.p35.linker.Δp40.

This plasmid was sequenced using the following primers: 5'-CAGAGTGAAAATGAAGCT-3' (SEQ ID NO: 18) and 5'-GAAGCTCTGCATCCTGCT-3' (SEQ ID NO: 19), corresponding to base pairs 601-618 and 613-630 of the IL-12p35 cDNA; and 5'-GTCATCTTCTTCAGGCGT-3' (SEQ ID NO: 34), an antisense primer corresponding to base pairs 217-200 of the IL-12 p40 cDNA. Sequencing confirmed that the sequence of the linker and contiguous IL-12 cDNA sequences were as expected.

### Plasmid pSFG. IL-12. p40. linker. ΔP35

The oligonucleotides were: sense, and antisense,

These two oligonucleotides were annealed, phosphorylated using T4 polynucleotide kinase, and ligated into the PflM1 site in pBS.IL-12.p35 which had been dephosphorylated using calf intestinal phosphatase. The orientation of this ligated fragment was confirmed by an Sse83871/EcoR1 digest. The Sse83871-EcoR1 fragment from the resultant plasmid was excised, the EcoR1 overhang having been filled using the Klenow fragment of *E*. *coli* DNA polymerase 1, and ligated into the Sse83871 and Klenow-filled BamH1 sites of pSFG.IL-12.p40 to create pSFG.IL-12.p4O.linker.Δp35.

This plasmid was sequenced using the primer 5'-GCAAAGGCGGGAATGTCT-3' (SEQ ID NO: 28), corresponding to base pairs 960-977 of the IL-12.p40 cDNA. The sequence of the second linker codon was difficult to read, but its sequence was determined by sequencing the cloned linker in the intermediate plasmid using the antisense primers
5'-AGGAATAATGTTTCAGTT-3' (SEQ ID NO: 29) and 5'-CAGCAGTGCAGGAATAAT-3' (SEQ ID NO: 30) corresponding to base pairs 224-207 and 233-216 of the IL-12 p35 cDNA respectively. Sequencing confirmed that the sequence of the linker and contiguous IL-12 cDNA sequences were as expected.

### Plasmids pSFG.IL-12.p35.IRES.p40 and pSFG.IL-12.p40.IRES.p35

The encephalomyelocarditis virus (ECMV) internal ribosome entry site (IRES) fragment was provided by Dr. Michael Sadelain (Whitehead Institute, Cambridge, MA), and was as previously described (Ghattas et al., Mol. Cell. Biol., 11:5848-5859 (1991)).

### EXAMPLE 2 Cells and Tissue Culture

BOSC23 packaging cells (Pear et al., Proc. Natl. Acad. Sci. USA. 90:8382-8396(1993)) were obtained from Dr. Dirk Lindemann (Whitehead Institute, Cambridge, MA). They were passaged in Dulbecco's modified Eagles medium (DMEM) supplemented with 10% calf serum, 50 U/ml penicillin and 50 µg/ml streptomycin.

CMS-5 tumor cells (DeLeo et al., J. Exp. Med., 146:720-734 (1977)) were obtained from Jason Salter (Whitehead Institute, Cambridge, MA). They were passaged in DMEM supplemented with 10% foetal calf serum, 50 U/ml penicillin and 50 pg/ml streptomycin. The same medium was used for the collection of CMS-5 conditioned medium.

C57BL/6 splenocytes for IL-12 assays were obtained by mincing a spleen through a sieve (Falcon 2350, Becton Dickinson, Franklin Lakes, NJ) and collecting the cells in IL-12 medium (as detailed in Schoenhaut et al. (J. Immunol., 148:3433-3440 (1992)) supplemented with 2% foetal calf serum.

### EXAMPLE 3 Generation of BOSC23-derived Producer Cells and Collection of Conditioned Media

BOSC23 cells were plated at 2 x 10⁶cells per 6 cm tissue culture dish and transfected by CaPO₄ transfection with the various constructs as previously described (Pear et al., Proc. Natl. Acad. Sci. USA, 90:8382-8396 (1993)). Twenty-four hours after transfection, the medium was replaced with 5 ml fresh medium. Virus-containing supernatants were collected 24 hours later, filtered through a 0.45 *µ*m filter and polybrene added to a final concentration of 8 *µ*g/ml. 2.5 ml of virus-containing supernatant was used to infect CMS-5 cells immediately for 4 hours (in preparation for this infection, CMS-5 cells had been plated at 5x10⁴ cells/6 cm tissue culture dish the previous day) and the remaining 2.5 ml frozen at -70 °C. The following day, the frozen 2.5 ml of virus-containing supernatant was thawed and used for a second 4 hour infection of the CMS-5 cells. To collect IL-12-containing conditioned medium, the medium was replaced the following day with 5 ml fresh medium which was harvested 24 hours later. These conditioned media were filtered through a 0.2 µm filter and frozen at -70°C for later assay for IL-12 bioactivity. 5 ml of fresh medium was added to the CMS-5 cells and a second set of conditioned media collected 24 hours later which were also filtered and frozen for later assay. The infected CMS-5 cells were then lysed, and genomic DNA prepared for later analysis.

### EXAMPLE 4 Bioassay for Murine Interleukin-12

Levels of bioactive interleukin-12 were determined using a concanavalin-A and interleukin-2 primed splenocyte proliferation assay, as described in Schoenhaut et al. (J. Immunol.,148:3433-3440 (1992)). The concanavalin A was obtained commercially from Boehringer (Mannheim, Germany) and the recombinant human interleukin-2 commercially from Chiron Therapeutics (Emeryville, CA). To harvest cells for the measurement of [³H]thymidine incorporation into cellular DNA, a Skatron (Sterling, VA) cell harvester and filtermats (#7031) were used. To assay for inhibitory activity in conditioned media, the 50 *µ*cl sample volume comprised 25 *µ*l of 1000 pg/ml recombinant murine IL-12 and 25 *µ*l of the test sample. Samples of conditioned media were assayed in duplicate at several dilutions in the range 1:1 to 1:1000. A standard curve was constructed for each bioassay using recombinant murine IL-12 in the range 20-10,000 pg/ml. The recombinant murine IL-12 was obtained from Hoffmann-La Roche (Nutley, NJ). To calculate the bioactive IL-12 concentration in test samples in pg/ml, the linear part of the standard curve was approximated using the curve-fit function of "KaleidaGraph 2.1.1" software and the resultant formula used for calculations. Conditioned media were verified to have hIL-12 immunoreactivity by hIL-12 ELISA assay (commercial kit, R & D Systems).

The following constructs (Figure 1) were assessed for their ability to express a bioactive IL-12 fusion protein:
A. pSFG.IL-12.p35.linker.p40;
B. pSFG.IL-12.p40.linker.p35;
C. pSFG.IL12-p35-linker.Δp40;
D. pSFG.IL12-p4C.linker.Δp35; and
E. pSFG.hIL-12.p40.1inker.Δp35.

The sequences for the linkers in each construct were as follows, as confirmed by sequencing (some adjacent confirmed IL-12 sequences are given for orientation):
A.
B.
C.
D. and
E.

No IL-12 bioactivity was detectable in media conditioned by mock-transfected CMS-5 cells, and CMS-5 cells infected with the SFG retrovirus alone, or by a related retrovirus (MFG) carrying the *lac-z* gene. However, media conditioned by these cells contained significant inhibitory activity at 1:2 and 1:10 dilutions, inhibiting as much as 95% of the bioactivity of 500 pg/ml of rmIL-12 (Table 1, and other data not shown). Despite this background of inhibitory activity in the conditioned media, bioactive IL-12 production proved to be still demonstrable.

Constructs for the expression of single subunits of the IL-12 protein (pSFG.Il-12.p35 and pSFG.IL-12.p40) resulted in no detectable bioactivity on their own. However, cotransfection of BOSC23 cells with these constructs together resulted in bioactive IL-12 secretion by infected CMS-5 cells. Similarly, CMS-5 cells infected with the SFG.IL-12.p35 retrovirus and 24 hours later with the SFG.IL-12.p40 retrovirus also produced bioactive IL-12 (Table 1).

The dicistronic constructs designed to express both IL-12 subunits using the IRES sequence resulted in similar levels of bioactive IL-12 production (despite an undetectable level of viral infection as determined by Southern hybridization analysis (see below)) (Table 1). The ability of IRES-containing retroviruses to result in bioactive IL-12 production has been confirmed by generating stable clonal retrovirus producing cell lines using both these constructs.

All IL-12 fusion protein constructs resulted in significant bioactive IL-12 production by infected CMS-5 cells. Of particular note was the SFG.IL-12.p40 linker.Δp35 construct, for which IL-12 bioactivity was demonstrable in undiluted conditioned medium (despite the background of substantial inhibitory activity) and for which a 1:1000 dilution of conditioned medium contained bioactivity equivalent to 301 pg/ml of rmIL-12 (Table 1). All constructs resulted in titratable IL-12 bioactivity despite significant non-specific inhibitory activity in the conditioned media as well. Bioactivity of hIL-12 was confirmed at Hoffman-LaRoche (Nutley, NJ, laboratory of Dr. M. Gately), and the specific activity of the hIL-12 fusion protein was determined to be approximately equivalent to the specific activity of recombinant native human IL-12.

**TABLE 1**

| Construct | Agonist assay (IL-12 bioactivity, pg/ml | | | Antagonist assay (% inhibition of 500 pg/ml IL-12 in assay) | | |
|---|---|---|---|---|---|---|
| | Dilution of CM in assay | | | Dilution of CM in assay | | |
| | 1:1 | 1:100 | 1:1000 | 1:2 | 1:10 | 1:1000 |
| No DNA | <50 | <50 | <50 | 56 | 62 | 8.6 |
| SFG-empty | <50 | <50 | <50 | 12 | 47 | -91 |
| MFG-lac-z | <50 | <50 | <50 | 66 | 56 | 64 |
| SFG.IL-12p35 | <50 | <50 | <50 | 65 | 76 | 45 |
| SFG.IL-12p40 | <50 | <50 | <50 | 94 | 84 | 38 |
| 2X infection^{a} | 199.7 | 234.2 | 137.2 | 1 | 1 | -84 |
| 2X transfection^{b} | 244.7 | 118.8 | <50 | 12 | -3 | -2 |
| A | 86.5 | <50 | <50 | 44 | 60 | 46 |
| B | 253.8 | <50 | <50 | 41 | 12 | -14 |
| C | 189.2 | 57.0 | <50 | 43 | 42 | 47 |
| D | 297.8 | 600.1 | 301.2 | -48 | -143 | -93 |

| | | | | | | |
|---|---|---|---|---|---|---|
| These data are from one of three separate assays. a. Target cells infected sequentially with pSFG.IL-12.p35 and then pSFG.IL-12.p40 viruses (each containing only the respective cDNA between the Ncol and BamH1 sites) b. BOSC23 cells were transfected with a mixture of pSFG.IL-12p35 and pSFG.IL-12.p40 constructs | | | | | | |

These data indicate IL-12 agonist activity was present in media conditioned by cells infected with the fusion protein retroviral constructs. It is presumed that this results from bioactivity of secreted respective fusion proteins.

The fusion proteins were demonstrated to be present using Western blotting. Serum-free CM from wild-type CMS-5 cells or CMS-5 cells expressing native IL-12 or the IL-12 fusion protein (SFG. IL-12.p35.IRES.p40 or SFG.IL-12. p40. linker. Δp35) were collected, filtered (0.2 *µ*m) and stored at -70°C. The CMs were concentrated 20-30-fold, and 20 *µ*g total protein sample was run on 10% polyacrylamide gels with or without 10% β-mercaptoethanol. The primary antibody was a polyclonal goat anti-rmIL-12 antibody (gift of D. Presky, Hoffman-La Roche, NJ). The "Renaissance" detection system (NEN Dupont) was used. A preliminary analysis indicated a 4-fold greater signal resulted from CM containing the single chain IL-12 (SFG.IL-12. p40. linker. Δp35) fusion protein, and hence a 5 *µ*g total protein sample from this CM was loaded. The control lanes were CM from wild-type cells without or spiked with 50 ng rmIL-12.

### EXAMPLE 5 Southern Hybridization Analysis of Genomic DNA from Infected CMS-5 Cells

Southern hybridization analysis of genomic DNA from the populations of infected CMS-5 cells was performed to demonstrate the presence of a hybridizing band consistent with infection of these cells by retroviruses of the expected structure, and to determine the efficiency of viral infection (by determination of retroviral copy number by genome).

From these Nhel digests of genomic DNA, a hybridizing retrovirus-derived band of 985 bp plus the size of the insert cloned into the Ncol-BamHl sites of SFG was predicted (See Figure 1). The size of the various cloned fragments were: IL-12.p35 cDNA, 0.6 kb; IL-12.p40 cDNA, 1.0 kb; IRES, 0.7 kb; linker, 0.05 kb; the putative leader sequence deleted in two constructs was 0.066 bp.

The BOSC23 cell supernatants resulted in viral copy numbers of between 0.1 and 1.4 copies/genome (mostly 0.1-0.3 copies/genome) for all constructs except for the IRES-containing constructs, where no hybridizing band of the expected size (3.2 kb) was seen (Table 2).

Of particular note are the comparative results for the IL-12 fusion proteins retrovirus constructs in these populations of infected cells. Although the pSFG.IL-12.p35.linker.p40 retrovirus was present at 1.4 copies/genome, this corresponded with a relatively low level of bioactive IL-12 production (Table 2). However, the SFG.IL-12.p40 linker.Δp35 retrovirus resulted in a relatively high level of IL-12 bioactivity, although it was present at 0.2 copies/genome.

**TABLE 2: Retrovirus Copy Number in CMS-5 Cells Infected bv SFG.IL-12 Retroviruses**

| SFG.IL-12 construct | Retrovirus |
|---|---|
| containing: | copy number^{a} |
| Nil | 0 |
| IL-12.p35 | 0.1 |
| IL-12.p40 | 0.3 |
| Sequential infection (p35/p40) | 0.3/0.3 |
| Co-transfection (p35/p40) | 0.1/0.1 |
| IL-12.p35-IRES-p40 | <<0.1^{b} |
| IL-12.p40.IRES.p35 | <<0.1^{b} |
| IL-12.p35.linker.p40 | 1.4^{b} |
| IL-12.p40.linker.p35 | 0.1^{b} |
| IL-12.p35.1inker.Δp40 | 0.4^{b} |
| IL-12.p40.linker.Δp35 | 0.2^{b} |
| 1 copy control | 1.0^{b} |
| 0.1 copy control | 0.1^{b} |

| | |
|---|---|
| ^{a} Relative to a plasmid copy number control of 13.5 pg of pSFG.IL-12.p35 linker.p40, calculated to be equimolar to 1 copy/genome for 10 µg genomic DNA. ^{b} Mean of results from one Southern blot probed first with a p35 and then with a p40 radiolabelled probe. Relative intensity of signals was quantitated using a Fuji BAS-II phosphoimager. | |

### EXAMPLE 6 Comparison of Immunotherapy of Established Immunogenic CMS-5 Tumors with GM-CSF-secreting and IL-12-secreting Tumor Cells

### Cytokine-Secreting Tumor Cells

SFG retroviruses generated by CRE or CRIP packaging cell lines were used for the transduction of tumor cells. The amount of cytokine secreted in vitro by the tumor cells used in these studies were (in ng/ml/48h/10⁶ irradiated cells [all collected in 10 ml]): cells infected with CRIP-packaged SFG.GM-CSF, B16>250, CMS-5>250; cells infected with CRE-packaged SFG.p35.IRES.p40.IL-12, B16 1-3, CMS-5 60-400; cells infected with CRE-packaged SFG.IL-12. p40. linker. Δp35, CMS-5 490-950; cells infected with CRIP-packaged SFG. IL-12. p35. IRES. p40, B16 90; and cells infected with CRIP-packaged SFG.IL-12.p40.linker.Δp35, B16 170 and RENCA 45. The tumor cells were irradiated to prevent the formation of additional tumors therefrom after injection into mice, and cytokine secretion was characterized for the same irradiated cells. GM-CSF concentrations of conditioned media (CM) were determined by ELISA (Endogen, Cambridge) and IL-12 levels by a bioassay based on the proliferation of concanavalin-A and interleukin-2 primed splenocytes (Schoenhaut et al. J. Immunol 148:3433 (1992)).

In an initial procedure, fibrosarcoma tumors were initiated with 2 x 10⁵ CMS-5 tumor cells injected subcutaneously on the back of syngeneic BALB/C mice, and immunotherapy (irradiated wild-type, GM-CSF-secreting or IL-12-secreting tumor cells) commenced either 7 or 14 days later. In this experiment, mice were stratified into multiple groups of 5 to 10 mice that received either 1, 2 or 3 weekly doses of immunotherapy at either 1 x 10⁶ or 5 x 10⁶ cells/dose. However, the primary analysis was stratified only by the type of cells used as immunotherapy and the day on which treatment began, regardless of other scheduling variables.

Mice treated with irradiated IL-12-secreting tumor cells showed significantly better long-term tumor-free survival compared to untreated mice or mice treated with wild-type or GM-CSF-secreting tumor cells, for therapy schedules starting either 7 or 14 days after tumor challenge (Figures 7A and 7B, p<0.05 for all comparisons with IL-12-secreting tumor cell immunotherapy). When immunotherapy was commenced 7 days after tumor transplantation, much of the survival advantage of mice treated with IL-12-secreting tumor cells was due to prevention of the development of late tumors (Figure 7C, p<0.05 for all comparisons by the log rank test). When immunotherapy commenced 14 days after tumor transplantation, much of the survival advantage was due to the regression of established tumors (Figures 7C, 7D and 8, p<0.005 compared to mice receiving wild-type or GM-CSF-secreting tumor cell immunotherapy). The palpable tumors that regressed in mice receiving IL-12-secreting tumor cell immunotherapy range from 1 to 8.5 mm in average diameter, and became impalpable 20-43 days (median 30 days) after tumor transplantation. The number of animals per group were respectively: 7A and 7B, 137, 40, 40, 38; and 7C and 7D, 18, 39, 40, 40. P-values given are for the least significant difference between treatment with IL-12-secreting cells and other groups. To determine the overall effect, data were pooled from groups that received like-immunotherapy cells by several schedules.

Subgroup analyses of therapies commencing on day 14 suggested that superior survival from immunotherapy with IL-12-secreting tumor cells resulted from schedules with more than one weekly dose of immunotherapy (p=0.1), doses of 5 x 10⁶ rather than 1 x 10⁶ IL-12-secreting cells (p<0.02). Hence, in all subsequent experiments utilizing transduced tumor cells, immunotherapy regimens comprised the higher cell dose administered weekly for 4 weeks.

### Statistical Analyses

All analyses were conducted on the basis of the intention to treat at the time of the random allocation of mice to groups. Descriptive statistics were calculated for major endpoints. Except where otherwise stated, differences in the survival endpoint were evaluated by the Wilcoxon rank-sum test. For survival analyses, occasional deaths immediately after anaesthesia and treatment were treated as censored events. The chi-square test was used to measure the association of categorical variables. Where p-values summarize the comparisons between multiple groups, only the largest p-value is given. Analyses were conducted using JMP software on a Power Macintosch 6100/60 computer.

### EXAMPLE 7 Study of Mechanisms of IL-12-induced Tumor Regression and Improved Survival

In order to determine whether immunotherapy with IL-12-secreting tumor cells was effective against larger tumor burdens, tumors were established with 4 x 10⁵ tumor cells, which, compared with 2 x 10⁵ cells, resulted in higher tumor incidence (day 14 palpability rates of 98/100 vs. 83/100, respectively), larger mean tumor size (6.7±3.0 vs. 3.7±2.4 mm diameter at day 14, respectively) and shorter median survival without treatment (31 vs. 37 days). Following establishment of these larger tumors, 70% (7/10) of tumor-bearing mice treated with IL-12-secreting tumor cell immunotherapy from day 14 survived with complete tumor regression, compared with 0/10 mice treated with wild-type tumor cells (p≤0.001).

The administration of IL-12 systemically (intraperitoneally) to mice bearing tumors initiated by 2 x 10⁵ CMS-5 cells also resulted in the regression of established tumors (Figure 9) and improved survival (4/5 at 90 days for mice treated with 0.1 µg/d, compared to 0/5 for placebo-treated mice). For mice with tumors established from 2 x 10⁵ CMS-5 cells, an IL-12 dose of 0.1 *µ*g/d (4/5 survival) was superior to 1 (3/5 survival), 0.01 (1/5 survival) and 0.001 *µ*g/d, for regimens starting either 7 or 14 days after tumor transplantation.

It was therefore possible that the regression of tumors in mice receiving immunotherapy with IL-12-secreting cells was not dependent on the local release of IL-12 at the site of the irradiated tumor cells administered as immunotherapy, but rather on a systemic effect of IL-12. This was evaluated by comparing different schedules starting on day 14 which combined either wild-type or IL-12-secreting tumor cell immunotherapy and systemic therapy with either IL-12, placebo or nothing. In mice with tumors initiated by 2 x 10⁵ CMS-5 cells, there was a tendency for median and overall survival to be better for immunotherapy with IL-12-secreting tumor cells than for systemic IL-12 therapy (Figure 10A). This tendency was statistically significant for mice with tumors initiated by 4 x 10⁵ cells (Figure 10B, p=0.006 comparing groups receiving systemic IL-12 (either alone or in combination with wild-type cells) vs. mice receiving IL-12-secreting tumor cell immunotherapy (either alone or with systemic therapy with diluent)). Comparisons of smaller, uniformly-treated groups (n=10/group, tumors initiated by 4 x 10⁵ cells) indicated that combining administration of wild type cells with systemic IL-12 was not different to systemic IL-12 therapy alone (p=0.85) and appeared inferior to vaccination with IL-12-secreting tumor cells alone (p=0.04) or given with placebo systemic therapy (p=0.19).

### EXAMPLE 8 Antitumor Effect of IL-12 Fusion Protein

In the pre-existing CMS-5 tumor model, immunotherapy with CMS-5 tumor cells expressing the IL-12 fusion protein SFG.IL-12.p40.linker.Δp35 was as effective as therapy with tumor cells making native IL-12 (Figures 11A and 11B). For mice with tumors initiated by either 2 x 10⁵ or 4 x 10⁵ CMS-5, survival was greater than 90% for groups of mice treated with CMS-5 cells secreting either form of IL-12, compared to less than 40% for mice that received no treatment, or treatment with wild-type or GM-CSF-secreting cells (p ≤ 0.02).

### EXAMPLE 9 Comparison of Immunotherapy of Established Non-immunogenic B16 Tumors with GM-CSF-secretinq and IL-12-secreting Tumor Cells

In order to assess the efficacy of immunotherapy with IL-12-secreting tumor cells in another tumor model, the non-immunogenic B16 melanoma was studied. B16 tumor cells were transduced to make native IL-12 at 90 ng/ml/48hr/10⁶ irradiated cells or the single chain IL-12 (SFG.IL-12.p40.linker.Δp35) at 170 ng/ml/48hr/10⁶ irradiated cells. B16 tumors were initiated with 4 x 10⁵ cells and immunotherapy of established tumors commenced either on day 7 (25% tumor palpability) or day 14 (93% tumor palpability, mean tumor diameter 5.74±3.23, n=56)). This procedure was analyzed after 31 days of follow up, when only 1/60 (2%) of mice that were treated with wild-type cells, CM-CSF-secreting cells or nothing as immunotherapy survived. Although mice treated with IL-12-secreting cells had comparably poor overall survival, their median survival was significantly prolonged compared to that of control mice treated with wild-type cells when treatment commenced on day 7 (Figure 12A, 24 vs. 18 days p=0.01) and day 14 (Figure 12B, 28 vs. 18 days, p=0.0005). Similarly, median survival was prolonged with therapy with IL-12 fusion protein-secreting tumor cells when treatment commenced on day 7 (21 vs. 18 days, p=0.08) and day 14 (24 vs. 18 days, p=0.006). In 3/4 scenarios, IL-12-secreting tumor cells were superior to GM-CSF-secreting cells (respective p-values 0.01, 0.14, 0.003, 0.02).

Given the potent effect of GM-CSF-secreting B16 cells to induce antitumor immunity when used as a vaccine prior to tumor challenge, but their lack of effect on tumor growth when administered after tumor establishment, the effects of IL-12-secreting and GM-CSF-secreting B16 cells were compared as vaccines in a B16 tumor challenge model. The IL-12-secreting B16 cells used in initial studies secreted native IL-12 at 1-3 ng/ml/48hr/10⁶ irradiated cells. GM-CSF-secreting B16 cells induced antitumor immunity when used as vaccines before tumor transplantation (Figure 12C, 80% 100-day survival).

### EXAMPLE 10 Immunotherapeutic Effect of IL-12 Delivery by Tumor Cells of Different Origin from Tumor to be Treated

The effect of the delivery of IL-12 by tumor cells of different origin from the tumor to be treated on survival was assessed in renal cell carcinoma (RENCA) tumors. RENCA tumors were initiated with 4 x 10⁵ cells, and immunotherapy of established tumors commenced on day 14. In one procedure, groups of mice were treated with either irradiated wild type CMS-5 cells or CMS-5 tumor cells transduced to secrete either native IL-12 or the fusion protein SFG.IL-12.p40.linker.Δp35 (Figure 13A). In another procedure, additional groups of mice were treated with a combination of irradiated CMS-5 and RENCA wild type cells, a combination of irradiated wild type CMS-5 and IL-12-secreting RENCA tumor cells or a combination of irradiated wild type RENCA tumor cells and IL-12-secreting CMS-5 tumor cells (Figure 13B).

In the first procedure, immunotherapy with both CMS-5 tumor cells secreting native IL-12 and the IL-12 fusion protein prolonged the median survival (p-values of p=0.02 and p=0.06, respectively). In the second procedure, mice treated with a combination of irradiated RENCA tumor cells and IL-12-secreting CMS-5 tumor cells exhibited a trend toward increased survival.

Additionally, CMS-5 tumors were initiated with 4 x 10⁵ cells, and immunotherapy of established tumors commenced on day 14. In one procedure, the established CMS-5 tumors were treated with either irradiated wild type RENCA tumor cells or RENCA tumor cells transduced to secrete either native IL-12 or the IL-12 fusion protein SFG.IL-12.p40.linker.Δp35 (Figure 14A). In another procedure, additional groups of mice were treated with a combination of irradiated CMS-5 and RENCA wild type cells, a combination of irradiated wild type CMS-5 and IL-12-secreting RENCA tumor cells or a combination of irradiated wild type RENCA tumor cells and IL-12-secreting CMS-5 tumor cells (Figure 14B).

In the first procedure, immunotherapy with RENCA cells secreting the IL-12 fusion protein moderately prolonged the survival of the mice compared to mice treated with wild type RENCA cells; the effect was consistent with the lower dose of IL-12 delivered by the transduced RENCA cells. In the second procedure, both mice treated with a combination of IL-12-secreting CMS-5 cells and wild type RENCA cells and mice treated with a combination of IL-12-secreting CMS-5 cells and wild type RENCA cells showed significantly prolonged survival (p-values of p=0.004 and p=0.04) compared with mice treated with wild type RENCA and wild type CMS-5 cells.

### Equivalents

Those skilled in the art will recognize, or be able to ascertain using no more than routine experimentation, many equivalents to the specific embodiments of the invention described herein. Such equivalents are intended to be encompassed by the following claims.

## Claims

1. DNA comprising DNA encoding IL-12 p35 subunit, DNA encoding a polypeptide linker and DNA encoding IL-12 p40 subunit, wherein the DNA encoding the polypeptide linker is positioned between the DNA encoding the IL-12 p35 subunit and the DNA encoding the IL-12 p40 subunit and wherein expression of the DNA results in production of a bioactive IL-12 fusion protein comprising the IL-12 p35 subunit and the IL-12 p40 subunit joined by the encoded polypeptide linker.

2. DNA of claim 1 wherein the IL-12 p35 subunit and the IL-12 p40 subunit are of human or mouse origin and the polypeptide linker is selected from the group consisting of: SEQ ID NOS: 5-7.

3. DNA of claim 1 encoding a bioactive IL-12 protein, wherein the bioactive IL-12 protein comprises native IL-12 p35 subunit and native IL-12 p40 subunit joined by a polypeptide linker.

4. DNA of claim 3 wherein the polypeptide linker is selected from the group consisting of: SEQ ID NOS: 5-7.

5. A bioactive IL-12 protein which comprises IL-12 p35 subunit and IL-12 p40 subunit joined by a polypeptide linker.

6. A bioactive IL-12 fusion protein according to claim 5 and encoded by the DNA of claim 1, 2 or 3.

7. A bioactive IL-12 protein of claim 5 wherein the IL-12 p35 subunit and the IL-12 p40 subunit are of human or mouse origin and the polypeptide linker is 7 to 16 amino acid residues.

8. A bioactive IL-12 protein of claim 7 wherein the polypeptide linker is selected from the group consisting of: SEQ ID NOS: 5-7.

9. An expression vector comprising DNA of claim 1.

10. An expression vector of claim 9 which is a retroviral vector.

11. An expression vector of claim 10 which comprises the pUC19-SFG retroviral backbone, as set out in Figure 3.

12. An expression vector of claim 11 comprising an insert between the Nco1 and BamH1 restriction enzyme sites of the pUC19-SFG retroviral backbone, encoding the following polypeptides: the IL-12 p35 subunit; a (Gly₄Ser)₂ Ser linker; and the IL-12 p40 subunit.

13. An expression vector of claim 11 comprising an insert, between the Nco1 and BamH1 restriction enzyme sites of the pUC19-SFG retroviral backbone, encoding the following polypeptides: the IL-12 p40 subunit; a (Gly₄Ser)₃ linker; and the IL-12p35 subunit.

14. An expression vector of claim 11 comprising an insert, between the Nco1 and BamH1 restriction enzyme sites of the pUC19-SFG retroviral backbone, encoding the following polypeptides: the IL-12 p35 subunit; a (Gly₄Ser)₃ linker; and the IL-12 p40 subunit with the first 22 amino acids deleted.

15. An expression vector of claim 11 comprising an insert between the Nco1 and BamH1 restriction enzyme sites of the pUC19-SFG retroviral backbone encoding the following peptides: the IL-12 p40 subunit; a (Gly₄Ser)₃ linker; and the IL-12 p35 subunit with first 22 amino acids deleted.

16. A method of producing a bioactive IL-1 2 protein comprising the steps of:
a) providing an expression vector comprising DNA encoding IL-12 p35 subunit, DNA encoding a polypeptide linker and DNA encoding IL-12 p40 subunit, wherein the DNA encoding the polypeptide linker is positioned between the DNA encoding the native IL-12 p35 subunit and the DNA encoding the native IL-12 p40 subunit;
b) introducing the expression vector into an appropriate host cell; and
c) maintaining the host cell resulting from step (b) under conditions appropriate for expression of the DNA present in the expression vector, resulting in production of a bioactive IL-12 protein in which the two subunits are joined by the polypeptide linker.

17. The method of claim 16, wherein the IL-12 p35 subunit and the IL-12 p40 subunit are of human or mouse origin and the polypeptide linker is 7 to 16 amino acid residues.

18. The method of claim 17, wherein the polypeptide linker is selected from the group consisting of: SEQ ID NOS: 5-7.

19. The method of claim 16, wherein the expression vector is a retroviral vector.

20. The method of claim 19, wherein the expression vector comprises the pUC19-SFG retroviral backbone, as set out in Figure 3.

21. The method of claim 20 comprising an insert, between the Nco1 and BamH1 restriction enzyme sites of the pUC19-SFG retroviral backbone, encoding the following polypeptides: the IL-12 p35 subunit; a (Gly₄Ser)₂ Ser linker; and the IL-12 p40 subunit.

22. The method of claim 20 comprising an insert, between the Nco1 and BamH1 restriction enzyme sites of the pUC19-SFG retroviral backbone, encoding the following polypeptides: the IL-12 p40 subunit; a (Gly₄Ser)₃ linker; and the IL-12p35 subunit.

23. The method of claim 20 comprising an insert, between the Nco1 and BamH1 restriction enzyme sites of the pUC19-SFG retroviral backbone, encoding the following polypeptides: the IL-12 p35 subunit; a (Gly₄Ser)₃ linker; and the IL-12 p40 subunit with the first 22 amino acids deleted.

24. The method of claim 20 comprising an insert, between the Nco1 and BamH1 l restriction enzyme sites of the pUC19-SFG retroviral backbone, encoding the following peptides: the IL-12 p40 subunit; a (Gly₄Ser)₃ linker; and the IL-12 p35 subunit with first 22 amino acids deleted.

## Patentansprüche

1. DNA, die aufweist: eine DNA, die eine IL-12 p35-Untereinheit codiert, eine DNA, die einen Polypeptidlinker codiert, und eine DNA die eine IL-12 p40-Untereinheit codiert, wobei die den Polypeptidlinker codierende DNA zwischen der die IL-12 p35-Untereinheit codierenden DNA und der die IL-12 p40-Untereinheit codierenden DNA positioniert ist, und wobei die Expression der DNA zur Erzeugung eines bioaktiven IL-12-Fusionsproteins führt, das die IL-12 p35-Untereinheit und die IL-12 p40-Untereinheit aufweist, die durch den codierten Polypeptidlinker miteinander verbunden sind.

2. DNA nach Anspruch 1, wobei die IL-12 p35-Untereinheit und die IL-12 p40-Untereinheit ihren Ursprung im Menschen oder der Maus haben und der Polypeptidlinker aus der Gruppe ausgewählt ist, die aus den SEQ ID-Nummern 5-7 besteht.

3. DNA nach Anspruch 1, die ein bioaktives IL-12-Protein codiert, wobei das bioaktive IL-12-Protein eine native IL-12 p35-Untereinheit und eine native IL-12 p40-Untereinheit aufweist, die durch einen Polypeptidlinker miteinander verbunden sind.

4. DNA nach Anspruch 3, wobei der Polypeptidlinker aus der Gruppe ausgewählt ist, die aus den SEQ ID-Nummern 5-7 besteht.

5. Bioaktives IL-12-Protein, das eine IL-12 p35-Untereinheit und eine IL-12 p40-Untereinheit aufweist, die durch einen Polypeptidlinker miteinander verbunden sind.

6. Bioaktives IL-12-Fusionsprotein nach Anspruch 5, das durch die DNA nach Anspruch 1, 2 oder 3 codiert wird.

7. Bioaktives IL-12-Protein nach Anspruch 5, wobei die IL-12 p35-Untereinheit und die IL-12 p40-Untereinheit ihren Ursprung im Menschen oder der Maus haben und der Polypeptidlinker aus 7 bis 16 Aminosäureresten besteht.

8. Bioaktives IL-12-Protein nach Anspruch 7, wobei der Polypeptidlinker aus der Gruppe ausgewählt ist, die aus den SEQ ID-Nummern 5-7 besteht.

9. Expressionsvektor, der die DNA nach Anspruch 1 aufweist.

10. Expressionsvektor nach Anspruch 9, der ein retroviraler Vektor ist.

11. Expressionsvektor nach Anspruch 10, der die retrovirale pUC19-SFG-Hauptkette aufweist, wie in Fig. 3 dargestellt.

12. Expressionsvektor nach Anspruch 11, der ein Insert zwischen den Nco1- und BamH1-Restriktionsenzymschnittstellen der retroviralen pUC19-SFG-Hauptkette aufweist, das die folgenden Polypeptide codiert: die IL-12 p35-Untereinheit; einen (Gly₄Ser)₂-Ser-Linker und die IL-12 p40-Untereinheit.

13. Expressionsvektor nach Anspruch 11, der ein Insert zwischen den Nco1- und BamH1-Restriktionsenzymschnittstellen der retroviralen pUC19-SFG-Hauptkette aufweist, das die folgenden Polypeptide codiert: die IL-12 p40-Untereinheit; einen (Gly₄Ser)₃-Linker und die IL-12-p35-Untereinheit.

14. Expressionsvektor nach Anspruch 11, der ein Insert zwischen den Nco1- und BamH1-Restriktionsenzymschnittstellen der retroviralen pUC19-SFG-Hauptkette aufweist, das die folgenden Polypeptide codiert: die IL-12 p35-Untereinheit ; einen (Gly₄Ser)₃-Linker und die IL-12 p40-Untereinheit, in der die ersten 22 Aminosäuren gestrichen sind.

15. Expressionsvektor nach Anspruch 11, der ein Insert zwischen den Nco1- und BamH1-Restriktionsenzymschnittstellen der retroviralen pUC19-SFG-Hauptkette aufweist, das die folgenden Peptide codiert: die IL-12 p40-Untereinheit ; einen (Gly₄Ser)₃-Linker und die IL-12 p35-Untereinheit, in der die ersten 22 Aminosäuren gestrichen sind.

16. Verfahren zur Herstellung eines bioaktiven IL-12-Proteins, das die folgenden Schritte aufweist:
a) Bereitstellen eines Expressionsvektors mit einer DNA, die eine IL-12 p35-Untereinheit codiert, einer DNA, die einen Polypeptidlinker codiert, und einer DNA, die eine IL-12 p40-Untereinheit codiert, wobei die den Polypeptidlinker codierende DNA zwischen der die native IL-12 p35-Untereinheit codierenden DNA und der die native IL-12 p40-Untereinheit codierenden DNA positioniert ist;
b) Einbringen des Expressionsvektors in eine geeignete Wirtszelle; und
c) Halten der aus Schritt (b) entstehenden Wirtszelle unter Bedingungen, die sich für eine Expression der in dem Expressionsvektor vorhandenen DNA eignen, wodurch ein bioaktives IL-12-Protein erzeugt wird, in dem die zwei Untereinheiten durch den Polypeptidlinker verbunden sind.

17. Verfahren nach Anspruch 16, wobei die IL-12 p35-Untereinheit und die IL-12 p40-Untereinheit ihren Ursprung im Menschen oder der Maus haben und der Polypeptidlinker aus 7 bis 16 Aminosäureresten besteht.

18. Verfahren nach Anspruch 17, wobei der Polypeptidlinker aus der Gruppe ausgewählt ist, die aus den SEQ ID-Nummern 5-7 besteht.

19. Verfahren nach Anspruch 16, wobei der Expressionsvektor ein retroviraler Vektor ist.

20. Verfahren nach Anspruch 19, wobei der Expressionsvektor die retrovirale pUC19-SFG-Hauptkette aufweist, wie in Fig. 3 dargestellt.

21. Verfahren nach Anspruch 20, das ein Insert zwischen den Nco1- und BamH1-Restriktionsenzymschnittstellen der retroviralen pUC19-SFG-Hauptkette aufweist, das die folgenden Polypeptide codiert: die IL-12 p35-Untereinheit; einen (Gly₄Ser)₂-Ser-Linker; und die IL-12 p40-Untereinheit.

22. Verfahren nach Anspruch 20, das ein Insert zwischen den Nco1- und BamH1-Restriktionsenzymschnittstellen der retroviralen pUC19-SFG-Hauptkette aufweist, das die folgenden Polypeptide codiert: die IL-12 p40-Untereinheit ; einen (Gly₄Ser)₃-Linker; und die IL-12 p35-Untereinheit.

23. Verfahren nach Anspruch 20, das ein Insert zwischen den Nco1- und BamH1-Restriktionsenzymschnittstellen der retroviralen pUC19-SFG-Hauptkette aufweist, das die folgenden Polypeptide codiert: die IL-12 p35-Untereinheit ; einen (Gly₄Ser)₃-Linker; und die IL-12 p40-Untereinheit, in der die ersten 22 Aminosäuren gestrichen sind.

24. Verfahren nach Anspruch 20, das ein Insert zwischen den Nco1- und BamH1-Restriktionsenzymschnittstellen der retroviralen pUC19-SFG-Hauptkette aufweist, das die folgenden Peptide codiert: die IL-12 p40-Untereinheit ; einen (Gly₄Ser)₃-Linker; und die IL-12 p35-Untereinheit, in der die ersten 22 Aminosäuren gestrichen sind.

## Revendications

1. ADN comprenant un ADN codant une sous-unité p35 d'IL-12, un ADN codant un adaptateur polypeptidique et un ADN codant une sous-unité p40 d'IL-12, dans lequel l'ADN codant l'adaptateur polypeptidique est situé entre l'ADN codant la sous-unité p35 d'IL-12 et l'ADN codant la sous-unité p40 d'IL-12, et en quoi l'expression de l'ADN a pour résultat la production d'une protéine de fusion d'IL-12 bioactive comprenant la sous-unité p35 d'IL-12 et la sous-unité p40 d'IL-12 liées par l'adaptateur polypeptidique codé.

2. ADN selon la revendication 1 dans lequel la sous-unité p35 d'IL-12 et la sous-unité p40 d'IL-12 sont d'origine humaine ou murine et l'adaptateur polypeptidique est choisi dans le groupe comprenant les Séquences n° 5 à 7.

3. ADN selon la revendication 1 codant une protéine d'IL-12 bioactive, en quoi la protéine d'IL-1 bioactive comprend une sous-unité p35 d'IL-12 naturelle et une sous-unité p40 d'IL-12 naturelle liées par un adaptateur polypeptidique.

4. ADN selon la revendication 3 dans lequel l'adaptateur polypeptidique est choisi dans le groupe comprenant les Séquences n° 5 à 7.

5. Protéine d'IL-12 bioactive qui comprend une sous-unité p35 d'IL-12 et une sous-unité p40 d'IL-12 liées par un adaptateur polypeptidique.

6. Protéine de fusion d'IL-12 bioactive conforme à la revendication 5 et codée par l'ADN selon la revendication 1, 2 ou 3.

7. Protéine d'IL-12 bioactive selon la revendication 5 dans laquelle la sous-unité p35 d'IL-12 et la sous-unité p40 d'IL-12 sont d'origine humaine ou murine et l'adaptateur polypeptidique est de 7 à 16 résidus d'acides aminés.

8. Protéine d'IL-12 bioactive selon la revendication 7 dans laquelle l'adaptateur polypeptidique est choisi dans le groupe comprenant les Séquences n° 5 à 7.

9. Vecteur d'expression comprenant l'ADN selon la revendication 1.

10. Vecteur d'expression selon la revendication 9 qui est vecteur rétroviral.

11. Vecteur d'expression selon la revendication 10 qui comprend le squelette rétroviral de pUC19-SFG, comme exposé en Figure 3.

12. Vecteur d'expression selon la revendication 11 comprenant un insert entre les sites des enzymes de restriction Nco1 et BamH1 du squelette rétroviral de pUC19-SFG, codant les polypeptides suivants: la sous-unité p35 d'IL-12; un adaptateur (Gly₄Ser)₂ Ser; et la sous-unité p40 d'IL-12.

13. Vecteur d'expression selon la revendication 11 comprenant un insert entre les sites des enzymes de restriction Nco1 et BamH1 du squelette rétroviral de pUC19-SFG, codant les polypeptides suivants: la sous-unité p40 d'IL-12; un adaptateur (Gly₄Ser)₃; et la sous-unité p35 d'IL-12.

14. Vecteur d'expression selon la revendication 11 comprenant un insert entre les sites des enzymes de restriction Nco1 et BamH1 du squelette rétroviral de pUC19-SFG, codant les polypeptides suivants: la sous-unité p35 d'IL-12; un adaptateur (Gly₄Ser)₃; et la sous-unité p40 d'IL-12 avec les 22 premiers acides aminés supprimés.

15. Vecteur d'expression selon la revendication 11 comprenant un insert entre les sites des enzymes de restriction Nco1 et BamH1 du squelette rétroviral de pUC19-SFG, codant les polypeptides suivants: la sous-unité p40 d'IL-12; un adaptateur (Gly₄Ser)₃; et la sous-unité p35 d'IL-12 avec les 22 premiers acides aminés supprimés.

16. Procédé de production d'une protéine d'IL-12 bioactive comprenant les étapes consistant à:
a) fournir un vecteur d'expression comprenant un ADN codant une sous-unité p35 d'IL-12, un ADN codant un adaptateur polypeptidique et un ADN codant une sous-unité p40 d'IL-12, dans lequel l'ADN codant l'adaptateur polypeptidique est situé entre l'ADN codant la sous-unité p35 d'IL-12 naturelle et l'ADN codant la sous-unité p40 d'IL-12 naturelle;
b) introduire le vecteur d'expression dans une cellule hôte adaptée; et
c) maintenir la cellule hôte résultant de l'étape (b) en conditions adaptées à l'expression de l'ADN présent dans le vecteur d'expression, ayant pour résultat la production d'une protéine d'IL-12 bioactive dans laquelle les deux sous-unités sont liées par l'adaptateur polypeptidique.

17. Procédé selon la revendication 16 dans lequel la sous-unité p35 d'IL-12 et la sous-unité p40 d'IL-12 sont d'origine humaine ou murine et l'adaptateur polypeptidique est de 7 à 16 résidus d'acides aminés.

18. Procédé selon la revendication 17 dans lequel l'adaptateur polypeptidique est choisi dans le groupe comprenant les Séquences n° 5 à 7.

19. Procédé selon la revendication 16 dans lequel le vecteur d'expression est un vecteur rétroviral.

20. Procédé selon la revendication 19 dans lequel le vecteur d'expression comprend le squelette rétroviral de pUC19-SFG, comme exposé en Figure 3.

21. Procédé selon la revendication 20 comprenant un insert entre les sites des enzymes de restriction Nco1 et BamHl du squelette rétroviral de pUC19-SFG, codant les polypeptides suivants: la sous-unité p35 d'IL-12; un adaptateur (Gly₄Ser)₂ Ser; et la sous-unité p40 d'IL-12.

22. Procédé selon la revendication 20 comprenant un insert entre les sites des enzymes de restriction Nco1 et BamHl du squelette rétroviral de pUC19-SFG, codant les polypeptides suivants: la sous-unité p40 d'IL-12; un adaptateur (Gly₄Ser)₃; et la sous-unité p35 d'IL-12.

23. Procédé selon la revendication 20 comprenant un insert entre les sites des enzymes de restriction Nco1 et BamHl du squelette rétroviral de pUC19-SFG, codant les polypeptides suivants: la sous-unité p35 d'IL-12; un adaptateur (Gly₄Ser)₃; et la sous-unité p40 d'IL-12 avec les 22 premiers acides aminés supprimés.

24. Procédé selon la revendication 20 comprenant un insert entre les sites des enzymes de restriction Nco1 et BamHl du squelette rétroviral de pUC19-SFG, codant les polypeptides suivants: la sous-unité p40 d'IL-12; un adaptateur (Gly₄Ser)₃; et la sous-unité p35 d'IL-12 avec les 22 premiers acides aminés supprimés.


### SEQUENCE LISTING

(1) GENERAL INFORMATION:
   (i) APPLICANT:
      (A) NAME: Whitehead Institute for Biomedical Research
      (B) STREET: 9 Cambridge Center
      (C) CITY: Cambridge
      (D) STATE/PROVINCE: MA
      (E) COUNTRY: US
      (F) POSTAL CODE/ZIP: 02142
      (G) TELEPHONE: 617-258-5104
      (I) TELEFAX: 617-258-6294

   (i) APPLICANT/INVENTOR:
      (A) NAME: Graham J. Lieschke
      (B) STREET: 5 Rollins Court
      (C) CITY: Cambridge
      (D) STATE/PROVINCE: MA
      (E) COUNTRY: US
      (F) POSTAL CODE/ZIP: 02139

   (i) APPLICANT/INVENTOR:
      (A) NAME: Richard C. Mulligan
      (B) STREET: 2 Sandy Pond Road
      (C) CITY: Lincoln
      (D) STATE/PROVINCE: MA
      (E) COUNTRY: US
      (F) POSTAL CODE/ZIP: 01773
   (ii) TITLE OF INVENTION: Bioactive Fusion Proteins and Pre-existing Tumor Therapy
   (iii) NUMBER OF SEQUENCES: 36
   (iv) CORRESPONDENCE ADDRESS:
      (A) ADDRESSEE: Hamilton, Brook, Smith & Reynolds, P.C.
      (B) STREET: Two Militia Drive
      (C) CITY: Lexington
      (D) STATE: Massachusetts
      (E) COUNTRY: USA
      (F) ZIP: 02173
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: Patent In Release #1.0, Version #1.30
   (vi) CURRENT APPLICATION DATA:
      (A) APPLICATION NUMBER:
      (B) FILING DATE:
      (C) CLASSIFICATION:
   (vii) PRIOR APPLICATION DATA:
      (A) APPLICATION NUMBER: US 08/385,335
      (B) FILING DATE: 08-FEB-1995
   (viii) ATTORNEY/AGENT INFORMATION:
      (A) NAME: Granahan, Patricia
      (B) REGISTRATION NUMBER: 32,227
      (C) REFERENCE/DOCKET NUMBER: WHI95-01A.PCT
   (ix) TELECOMMUNICATION INFORMATION:
      (A) TELEPHONE: 617-861-6240
      (B) TELEFAX: 617-861-9540
(2) INFORMATION FOR SEQ ID NO:1:
   (i) SEQUENCE CHARACTERISTICS:
      (A) LENGTH: 54 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:1:
      AGCTCCGCCG GTGGTGGTGG GTCGGGTGGC GGCGGATCTT CCATGGGTCC TCAG 54
(2) INFORMATION FOR SEQ ID NO:2:
   (i) SEQUENCE CHARACTERISTICS:
      (A) LENGTH: 66 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:2:
   GTCCGATCCG GTGGCGGTGG CTCGGGCGGT GGTGGGTCGG GTGGCGGCGG ATCTTCCATG 60
   GGTCAA 66
(2) INFORMATION FOR SEQ ID NO:3:
   (i) SEQUENCE CHARACTERISTICS:
      (A) LENGTH: 66 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:3:
   AGCTCCGCCG GTGGCGGTGG CTCGGGCGGT GGTGGGTCGG GTGGCGGCGG ATCTATGTGG 60
   GAGCTG 66
(2) INFORMATION FOR SEQ ID NO:4:
   (i) SEQUENCE CHARACTERISTICS:
      (A) LENGTH: 66 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:4:
   GTCCGATCCG GTGGCGGTGG CTCGGGCGGT GGTGGGTCGG GTGGCGGCGG ATCTAGGGTC 60
   ATTCCA 66
(2) INFORMATION FOR SEQ ID NO:5:
   (i) SEQUENCE CHARACTERISTICS:
      (A) LENGTH: 11 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:5:
   Gly Gly Gly Gly Ser Gly Gly Gly Gly Ser Ser
   1 5 10
(2) INFORMATION FOR SEQ ID NO:6:
   (i) SEQUENCE CHARACTERISTICS:
      (A) LENGTH: 16 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:6:
   Gly Gly Gly Gly Ser Gly Gly Gly Gly Ser Gly Gly Gly Gly Ser Ser
   1 5 10 15
(2) INFORMATION FOR SEQ ID NO:7:
   (i) SEQUENCE CHARACTERISTICS:
      (A) LENGTH: 15 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:7:
   Gly Gly Gly Gly Ser Gly Gly Gly Gly Ser Gly Gly Gly Gly Ser
   1 5 10 15
(2) INFORMATION FOR SEQ ID NO:8:
   (i) SEQUENCE CHARACTERISTICS:
      (A) LENGTH: 6350 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:8:
   AAGCTTTGCT CTTAGGAGTT TCCTAATACA TCCCAAACTC AAATATATAA AGCATTTGAC 60
   TTGTTCTATG CCCTAGGGGG CGGGGGGAAG CTAAGCCAGC TTTTTTTAAC ATTTAAAATG 120
   TTAATTCCAT TTTAAATGCA CAGATGTTTT TATTTCATAA GGGTTTCAAT GTGCATGAAT 180
   GCTGCAATAT TCCTGTTACC AAAGCTAGTA TAAATAAAAA TAGATAAACG TGGAAATTAC 240
   TTAGAGTTTC TGTCATTAAC GTTTCCTTCC TCAGTTGACA ACATAAATGC GCTGCTGAGC 300
   AAGCCAGTTT GCATCTGTCA GGATCAATTT CCCATTATGC CAGTCATATT AATTACTAGT 360
   CAATTAGTTG ATTTTTATTT TTGACATATA CATGTGAATG AAAGACCCCA CCTGTAGGTT 420
   TGGCAAGCTA GCTTAAGTAA CGCCATTTTG CAAGGCATGG AAAAATACAT AACTGAGAAT 480
   AGAAAAGTTC AGATCAAGGT CAGGAACAGA TGGAACAGCT GAATATGGGC CAAACAGGAT 540
   ATCTGTGGTA AGCAGTTCCT GCCCCGGCTC AGGGCCAAGA ACAGATGGAA CAGCTGAATA 600
   TGGGCCAAAC AGGATATCTG TGGTAAGCAG TTCCTGCCCC GGCTCAGGGC CAAGAACAGA 660
   TGGTCCCCAG ATGCGGTCCA GCCCTCAGCA GTTTCTAGAG AACCATCAGA TGTTTCCAGG 720
   GTGCCCCAAG GACCTGAAAT GACCCTGTGC CTTATTTGAA CTAACCAATC AGTTCGCTTC 780
   TCGCTTCTGT TCGCGCGCTT ATGCTCCCCG AGCTCAATAA AAGAGCCCAC AACCCCTCAC 840
   TCGGGGCGCC AGTCCTCCGA TTGACTGAGT CGCCCGGGTA CCCGTGTATC CAATAAACCC 900
   TCTTGCAGTT GCATCCGACT TGTGGTCTCG CTGTTCCTTG GGAGGGTCTC CTCTGAGTGA 960
   TTGACTACCC GTCAGCGGGG GTCTTTCATT TGGGGGCTCG TCCGGGATCG GGAGACCCCT 1020
   GCCCAGGGAC CACCGACCCA CCACCGGGAG GTAAGCTGGC CAGCAACTTA TCTGTGTCTG 1080
   TCCGATTGTC TAGTGTCTAT GACTGATTTT ATGCGCCTGC GTCGGTACTA GTTAGCTAAC 1140
   TAGCTCTGTA TCTGGCGGAC CCGTGGTGGA ACTGACGAGT TCGGAACACC CGGCCGCAAC 1200
   CCTGGGAGAC GTCCCAGGGA CTTCGGGGGC CGTTTTTGTG GCCCGACCTG AGTCCTAAAA 1260
   TCCCGATCGT TTAGGACTCT TTGGTGCACC CCCCTTAGAG GAGGGATATG TGGTTCTGGT 1320
   AGGAGACGAG AACCTAAAAC AGTTCCCGCC TCCGTCTGAA TTTTTGCTTT CGGTTTGGGA 1380
   CCGAAGCCGC GCCGCGCGTC TTGTCTGCTG CAGCATCGTT CTGTGTTGTC TCTGTCTGAC 1440
   TGTGTTTCTG TATTTGTCTG AAAATATGGG CCCGGGCTAG CCTGTTACCA CTCCCTTAAG 1500
   TTTGACCTTA GGTCACTGGA AAGATGTCGA GCGGATCGCT CACAACCAGT CGGTAGATGT 1560
   CAAGAAGAGA CGTTGGGTTA CCTTCTGCTC TGCAGAATGG CCAACCTTTA ACGTCGGATG 1620
   GCCGCGAGAC GGCACCTTTA ACCGAGACCT CATCACCCAG GTTAAGATCA AGGTCTTTTC 1680
   ACCTGGCCCG CATGGACACC CAGACCAGGT GGGGTACATC GTGACCTGGG AAGCCTTGGC 1740
   TTTTGACCCC CCTCCCTGGG TCAAGCCCTT TGTACACCCT AAGCCTCCGC CTCCTCTTCC 1800
   TCCATCCGCC CCGTCTCTCC CCCTTGAACC TCCTCGTTCG ACCCCGCCTC GATCCTCCCT 1860
   TTATCCAGCC CTCACTCCTT CTCTAGGCGC CCCCATATGG CCATATGAGA TCTTATATGG 1920
   GGCACCCCCG CCCCTTGTAA ACTTCCCTGA CCCTGACATG ACAAGAGTTA CTAACAGCCC 1980
   CTCTCTCCAA GCTCACTTAC AGGCTCTCTA CTTAGTCCAG CACGAAGTCT GGAGACCTCT 2040
   GGCGGCAGCC TACCAAGAAC AACTGGACCG ACCGGTGGTA CCTCACCCTT ACCGAGTCGG 2100
   CGACACAGTG TGGGTCCGCC GACACCAGAC TAAGAACCTA GAACCTCGCT GGAAAGGACC 2160
   TTACACAGTC CTGCTGACCA CCCCCACCGC CCTCAAAGTA GACGGCATCG CAGCTTGGAT 2220
   ACACGCCGCC CACGTGAAGG CTGCCGACCC CGGGGGTGGA CCATCCTCTA GACTGCCATG 2280
   GCGCGGATCC GGATTAGTCC AATTTGTTAA AGACAGGATA TCAGTGGTCC AGGCTCTAGT 2340
   TTTGACTCAA CAATATCACC AGCTGAAGCC TATAGAGTAC GAGCCATAGA TAAAATAAAA 2400
   GATTTTATTT AGTCTCCAGA AAAAGGGGGG AATGAAAGAC CCCACCTGTA GGTTTGGCAA 2460
   GCTAGCTTAA GTAACGCCAT TTTGCAAGGC ATGGAAAAAT ACATAACTGA GAATAGAGAA 2520
   GTTCAGATCA AGGTCAGGAA CAGATGGAAC AGCTGAATAT GGGCCAAACA GGATATCTGT 2580
   GGTAAGCAGT TCCTGCCCCG GCTCAGGGCC AAGAACAGAT GGAACAGCTG AATATGGGCC 2640
   AAACAGGATA TCTGTGGTAA GCAGTTCCTG CCCCGGCTCA GGGCCAAGAA CAGATGGTCC 2700
   CCAGATGCGG TCCAGCCCTC AGCAGTTTCT AGAGAACCAT CAGATGTTTC CAGGGTGCCC 2760
   CAAGGACCTG AAATGACCCT GTGCCTTATT TGAACTAACC AATCAGTTCG CTTCTCGCTT 2820
   CTGTTCGCGC GCTTCTGCTC CCCGAGCTCA ATAAAAGAGC CCACAACCCC TCACTCGGGG 2880
   CGCCAGTCCT CCGATTGACT GAGTCGCCCG GGTACCCGTG TATCCAATAA ACCCTCTTGC 2940
   AGTTGCATCC GACTTGTGGT CTCGCTGTTC CTTGGGAGGG TCTCCTCTGA GTGATTGACT 3000
   ACCCGTCAGC GGGGGTCTTT CACACATGCA GCATGTATCA AAATTAATTT GGTTTTTTTT 3060
   CTTAAGTATT TACATTAAAT GGCCATAGTA CTTAAAGTTA CATTGGCTTC CTTGAAATAA 3120
   ACATGGAGTA TTCAGAATGT GTCATAAATA TTTCTAATTT TAAGATAGTA TCTCCATTGG 3180
   CTTTCTACTT TTTCTTTTAT TTTTTTTTGT CCTCTGTCTT CCATTTGTTG TTGTTGTTGT 3240
   TTGTTTGTTT GTTTGTTGGT TGGTTGGTTA ATTTTTTTTT AAAGATCCTA CACTATAGTT 3300
   CAAGCTAGAC TATTAGCTAC TCTGTAACCC AGGGTGACCT TGAAGTCATG GGTAGCCTGC 3360
   TGTTTTAGCC TTCCCACATC TAAGATTACA GGTATGAGCT ATCATTTTTG GTATATTGAT 3420
   TGATTGATTG ATTGATGTGT GTGTGTGTGA TTGTGTTTGT GTGTGTGACT GTGAAAATGT 3480
   GTGTATGGGT GTGTGTGAAT GTGTGTATGT ATGTGTGTGT GTGAGTGTGT GTGTGTGTGT 3540
   GTGCATGTGT GTGTGTGTGA CTGTGTCTAT GTGTATGACT GTGTGTGTGT GTGTGTGTGT 3600
   GTGTGTGTGT GTGTGTGTGT GTGTGTTGTG AAAAAATATT CTATGGTAGT GAGAGCCAAC 3660
   GCTCCGGCTC AGGTGTCAGG TTGGTTTTTG AGACAGAGTC TTTCACTTAG CTTGGAATTC 3720
   ACTGGCCGTC GTTTTACAAC GTCGTGACTG GGAAAACCCT GGCGTTACCC AACTTAATCG 3780
   CCTTGCAGCA CATCCCCCTT TCGCCAGCTG GCGTAATAGC GAAGAGGCCC GCACCGATCG 3840
   CCCTTCCCAA CAGTTGCGCA GCCTGAATGG CGAATGGCGC CTGATGCGGT ATTTTCTCCT 3900
   TACGCATCTG TGCGGTATTT CACACCGCAT ATGGTGCACT CTCAGTACAA TCTGCTCTGA 3960
   TGCCGCATAG TTAAGCCAGC CCCGACACCC GCCAACACCC GCTGACGCGC CCTGACGGGC 4020
   TTGTCTGCTC CCGGCATCCG CTTACAGACA AGCTGTGACC GTCTCCGGGA GCTGCATGTG 4080
   TCAGAGGTTT TCACCGTCAT CACCGAAACG CGCGATGACG AAAGGGCCTC GTGATACGCC 4140
   TATTTTTATA GGTTAATGTC ATGATAATAA TGGTTTCTTA GACGTCAGGT GGCACTTTTC 4200
   GGGGAAATGT GCGCGGAACC CCTATTTGTT TATTTTTCTA AATACATTCA AATATGTATC 4260
   CGCTCATGAG ACAATAACCC TGATAAATGC TTCAATAATA TTGAAAAAGG AAGAGTATGA 4320
   GTATTCAACA TTTCCGTGTC GCCCTTATTC CCTTTTTTGC GGCATTTTGC CTTCCTGTTT 4380
   TTGCTCACCC AGAAACGCTG GTGAAAGTAA AAGATGCTGA AGATCAGTTG GGTGCACGAG 4440
   TGGGTTACAT CGAACTGGAT CTCAACAGCG GTAAGATCCT TGAGAGTTTT CGCCCCGAAG 4500
   AACGTTTTCC AATGATGAGC ACTTTTAAAG TTCTGCTATG TGGCGCGGTA TTATCCCGTA 4560
   TTGACGCCGG GCAAGAGCAA CTCGGTCGCC GCATACACTA TTCTCAGAAT GACTTGGTTG 4620
   AGTACTCACC AGTCACAGAA AAGCATCTTA CGGATGGCAT GACAGTAAGA GAATTATGCA 4680
   GTGCTGCCAT AACCATGAGT GATAACACTG CGGCCAACTT ACTTCTGACA ACGATCGGAG 4740
   GACCGAAGGA GCTAACCGCT TTTTTGCACA ACATGGGGGA TCATGTAACT CGCCTTGATC 4800
   GTTGGGAACC GGAGCTGAAT GAAGCCATAC CAAACGACGA GCGTGACACC ACGATGCCTG 4860
   TAGCAATGGC AACAACGTTG CGCAAACTAT TAACTGGCGA ACTACTTACT CTAGCTTCCC 4920
   GGCAACAATT AATAGACTGG ATGGAGGCGG ATAAAGTTGC AGGACCACTT CTGCGCTCGG 4980
   CCCTTCCGGC TGGCTGGTTT ATTGCTGATA AATCTGGAGC CGGTGAGCGT GGGTCTCGCG 5040
   GTATCATTGC AGCACTGGGG CCAGATGGTA AGCCCTCCCG TATCGTAGTT ATCTACACGA 5100
   CGGGGAGTCA GGCAACTATG GATGAACGAA ATAGACAGAT CGCTGAGATA GGTGCCTCAC 5160
   TGATTAAGCA TTGGTAACTG TCAGACCAAG TTTACTCATA TATACTTTAG ATTGATTTAA 5220
   AACTTCATTT TTAATTTAAA AGGATCTAGG TGAAGATCCT TTTTGATAAT CTCATGACCA 5280
   AAATCCCTTA ACGTGAGTTT TCGTTCCACT GAGCGTCAGA CCCCGTAGAA AAGATCAAAG 5340
   GATCTTCTTG AGATCCTTTT TTTCTGCGCG TAATCTGCTG CTTGCAAACA AAAAAACCAC 5400
   CGCTACCAGC GGTGGTTTGT TTGCCGGATC AAGAGCTACC AACTCTTTTT CCGAAGGTAA 5460
   CTGGCTTCAG CAGAGCGCAG ATACCAAATA CTGTCCTTCT AGTGTAGCCG TAGTTAGGCC 5520
   ACCACTTCAA GAACTCTGTA GCACCGCCTA CATACCTCGC TCTGCTAATC CTGTTACCAG 5580
   TGGCTGCTGC CAGTGGCGAT AAGTCGTGTC TTACCGGGTT GGACTCAAGA CGATAGTTAC 5640
   CGGATAAGGC GCAGCGGTCG GGCTGAACGG GGGGTTCGTG CACACAGCCC AGCTTGGAGC 5700
   GAACGACCTA CACCGAACTG AGATACCTAC AGCGTGAGCA TTGAGAAAGC GCCACGCTTC 5760
   CCGAAGGGAG AAAGGCGGAC AGGTATCCGG TAAGCGGCAG GGTCGGAACA GGAGAGCGCA 5820
   CGAGGGAGCT TCCAGGGGGA AACGCCTGGT ATCTTTATAG TCCTGTCGGG TTTCGCCACC 5880
   TCTGACTTGA GCGTCGATTT TTGTGATGCT CGTCAGGGGG GCGGAGCCTA TGGAAAAACG 5940
   CCAGCAACGC GGCCTTTTTA CGGTTCCTGG CCTTTTGCTG GCCTTTTGCT CACATGTTCT 6000
   TTCCTGCGTT ATCCCCTGAT TCTGTGGATA ACCGTATTAC CGCCTTTGAG TGAGCTGATA 6060
   CCGCTCGCCG CAGCCGAACG ACCGAGCGCA GCGAGTCAGT GAGCGAGGAA GCGGAAGAGC 6120
   GCCCAATACG CAAACCGCCT CTCCCCGCGC GTTGGCCGAT TCATTAATGC AGCTGGCACG 6180
   ACAGGTTTCC CGACTGGAAA GCGGGCAGTG AGCGCAACGC AATTAATGTG AGTTAGCTCA 6240
   CTCATTAGGC ACCCCAGGCT TTACACTTTA TGCTTCCGGC TCGTATGTTG TGTGGAATTG 6300
   TGAGCGGATA ACAATTTCAC ACAGGAAACA GCTATGACCA TGATTACGCC 6350
(2) INFORMATION FOR SEQ ID NO:9:
   (i) SEQUENCE CHARACTERISTICS:
      (A) LENGTH: 6350 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:9:
   TTCGAAACGA GAATCCTCAA AGGATTATGT AGGGTTTGAG TTTATATATT TCGTAAACTG    60
   AACAAGATAC GGGATCCCCC GCCCCCCTTC GATTCGGTCG AAAAAAATTG TAAATTTTAC   120
   AATTAAGGTA AAATTTACGT GTCTACAAAA ATAAAGTATT CCCAAAGTTA CACGTACTTA   180
   CGACGTTATA AGGACAATGG TTTCGATCAT ATTTATTTTT ATCTATTTGC ACCTTTAATG   240
   AATCTCAAAG ACAGTAATTG CAAAGGAAGG AGTCAACTGT TGTATTTACG CGACGACTCG   300
   TTCGGTCAAA CGTAGACAGT CCTAGTTAAA GGGTAATACG GTCAGTATAA TTAATGATCA   360
   GTTAATCAAC TAAAAATAAA AACTGTATAT GTACACTTAC TTTCTGGGGT GGACATCCAA   420
   ACCGTTCGAT CGAATTCATT GCGGTAAAAC GTTCCGTACC TTTTTATGTA TTGACTCTTA   480
   TCTTTTCAAG TCTAGTTCCA GTCCTTGTCT ACCTTGTCGA CTTATACCCG GTTTGTCCTA   540
   TAGACACCAT TCGTCAAGGA CGGGGCCGAG TCCCGGTTCT TGTCTACCTT GTCGACTTAT   600
   ACCCGGTTTG TCCTATAGAC ACCATTCGTC AAGGACGGGG CCGAGTCCCG GTTCTTGTCT   660
   ACCAGGGGTC TACGCCAGGT CGGGAGTCGT CAAAGATCTC TTGGTAGTCT ACAAAGGTCC   720
   CACGGGGTTC CTGGACTTTA CTGGGACACG GAATAAACTT GATTGGTTAG TCAAGCGAAG   780
   AGCGAAGACA AGCGCGCGAA TACGAGGGGC TCGAGTTATT TTCTCGGGTG TTGGGGAGTG   840
   AGCCCCGCGG TCAGGAGGCT AACTGACTCA GCGGGCCCAT GGGCACATAG GTTATTTGGG   900
   AGAACGTCAA CGTAGGCTGA ACACCAGAGC GACAAGGAAC CCTCCCAGAG GAGACTCACT 960
   AACTGATGGG CAGTCGCCCC CAGAAAGTAA ACCCCCGAGC AGGCCCTAGC CCTCTGGGGA 1020
   CGGGTCCCTG GTGGCTGGGT GGTGGCCCTC CATTCGACCG GTCGTTGAAT AGACACAGAC 1080
   AGGCTAACAG ATCACAGATA CTGACTAAAA TACGCGGACG CAGCCATGAT CAATCGATTG 1140
   ATCGAGACAT AGACCGCCTG GGCACCACCT TGACTGCTCA AGCCTTGTGG GCCGGCGTTG 1200
   GGACCCTCTG CAGGGTCCCT GAAGCCCCCG GCAAAAACAC CGGGCTGGAC TCAGGATTTT 1260
   AGGGCTAGCA AATCCTGAGA AACCACGTGG GGGGAATCTC CTCCCTATAC ACCAAGACCA 1320
   TCCTCTGCTC TTGGATTTTG TCAAGGGCGG AGGCAGACTT AAAAACGAAA GCCAAACCCT 1380
   GGCTTCGGCG CGGCGCGCAG AACAGACGAC GTCGTAGCAA GACACAACAG AGACAGACTG 1440
   ACACAAAGAC ATAAACAGAC TTTTATACCC GGGCCCGATC GGACAATGGT GAGGGAATTC 1500
   AAACTGGAAT CCAGTGACCT TTCTACAGCT CGCCTAGCGA GTGTTGGTCA GCCATCTACA 1560
   GTTCTTCTCT GCAACCCAAT GGAAGACGAG ACGTCTTACC GGTTGGAAAT TGCAGCCTAC 1620
   CGGCGCTCTG CCGTGGAAAT TGGCTCTGGA GTAGTGGGTC CAATTCTAGT TCCAGAAAAG 1680
   TGGACCGGGC GTACCTGTGG GTCTGGTCCA CCCCATGTAG CACTGGACCC TTCGGAACCG 1740
   AAAACTGGGG GGAGGGACCC AGTTCGGGAA ACATGTGGGA TTCGGAGGCG GAGGAGAAGG 1800
   AGGTAGGCGG GGCAGAGAGG GGGAACTTGG AGGAGCAAGC TGGGGCGGAG CTAGGAGGGA 1860
   AATAGGTCGG GAGTGAGGAA GAGATCCGCG GGGGTATACC GGTATACTCT AGAATATACC 1920
   CCGTGGGGGC GGGGAACATT TGAAGGGACT GGGACTGTAC TGTTCTCAAT GATTGTCGGG 1980
   GAGAGAGGTT CGAGTGAATG TCCGAGAGAT GAATCAGGTC GTGCTTCAGA CCTCTGGAGA 2040
   CCGCCGTCGG ATGGTTCTTG TTGACCTGGC TGGCCACCAT GGAGTGGGAA TGGCTCAGCC 2100
   GCTGTGTCAC ACCCAGGCGG CTGTGGTCTG ATTCTTGGAT CTTGGAGCGA CCTTTCCTGG 2160
   AATGTGTCAG GACGACTGGT GGGGGTGGCG GGAGTTTCAT CTGCCGTAGC GTCGAACCTA 2220
   TGTGCGGCGG GTGCACTTCC GACGGCTGGG GCCCCCACCT GGTAGGAGAT CTGACGGTAC 2280
   CGCGCCTAGG CCTAATCAGG TTAAACAATT TCTGTCCTAT AGTCACCAGG TCCGAGATCA 2340
   AAACTGAGTT GTTATAGTGG TCGACTTCGG ATATCTCATG CTCGGTATCT ATTTTATTTT 2400
   CTAAAATAAA TCAGAGGTCT TTTTCCCCCC TTACTTTCTG GGGTGGACAT CCAAACCGTT 2460
   CGATCGAATT CATTGCGGTA AAACGTTCCG TACCTTTTTA TGTATTGACT CTTATCTCTT 2520
   CAAGTCTAGT TCCAGTCCTT GTCTACCTTG TCGACTTATA CCCGGTTTGT CCTATAGACA 2580
   CCATTCGTCA AGGACGGGGC CGAGTCCCGG TTCTTGTCTA CCTTGTCGAC TTATACCCGG 2640
   TTTGTCCTAT AGACACCATT CGTCAAGGAC GGGGCCGAGT CCCGGTTCTT GTCTACCAGG 2700
   GGTCTACGCC AGGTCGGGAG TCGTCAAAGA TCTCTTGGTA GTCTACAAAG GTCCCACGGG 2760
   GTTCCTGGAC TTTACTGGGA CACGGAATAA ACTTGATTGG TTAGTCAAGC GAAGAGCGAA 2820
   GACAAGCGCG CGAAGACGAG GGGCTCGAGT TATTTTCTCG GGTGTTGGGG AGTGAGCCCC 2880
   GCGGTCAGGA GGCTAACTGA CTCAGCGGGC CCATGGGCAC ATAGGTTATT TGGGAGAACG 2940
   TCAACGTAGG CTGAACACCA GAGCGACAAG GAACCCTCCC AGAGGAGACT CACTAACTGA 3000
   TGGGCAGTCG CCCCCAGAAA GTGTGTACGT CGTACATAGT TTTAATTAAA CCAAAAAAAA 3060
   GAATTCATAA ATGTAATTTA CCGGTATCAT GAATTTCAAT GTAACCGAAG GAACTTTATT 3120
   TGTACCTCAT AAGTCTTACA CAGTATTTAT AAAGATTAAA ATTCTATCAT AGAGGTAACC 3180
   GAAAGATGAA AAAGAAAATA AAAAAAAACA GGAGACAGAA GGTAAACAAC AACAACAACA 3240
   AACAAACAAA CAAACAACCA ACCAACCAAT TAAAAAAAAA TTTCTAGGAT GTGATATCAA 3300
   GTTCGATCTG ATAATCGATG AGACATTGGG TCCCACTGGA ACTTCAGTAC CCATCGGACG 3360
   ACAAAATCGG AAGGGTGTAG ATTCTAATGT CCATACTCGA TAGTAAAAAC CATATAACTA 3420
   ACTAACTAAC TAACTACACA CACACACACT AACACAAACA CACACACTGA CACTTTTACA 3480
   CACATACCCA CACACACTTA CACACATACA TACACACACA CACTCACACA CACACACACA 3540
   CACGTACACA CACACACACT GACACAGATA CACATACTGA CACACACACA CACACACACA 3600
   CACACACACA CACACACACA CACACAACAC TTTTTTATAA GATACCATCA CTCTCGGTTG 3660
   CGAGGCCGAG TCCACAGTCC AACCAAAAAC TCTGTCTCAG AAAGTGAATC GAACCTTAAG 3720
   TGACCGGCAG CAAAATGTTG CAGCACTGAC CCTTTTGGGA CCGCAATGGG TTGAATTAGC 3780
   GGAACGTCGT GTAGGGGGAA AGCGGTCGAC CGCATTATCG CTTCTCCGGG CGTGGCTAGC 3840
   GGGAAGGGTT GTCAACGCGT CGGACTTACC GCTTACCGCG GACTACGCCA TAAAAGAGGA 3900
   ATGCGTAGAC ACGCCATAAA GTGTGGCGTA TACCACGTGA GAGTCATGTT AGACGAGACT 3960
   ACGGCGTATC AATTCGGTCG GGGCTGTGGG CGGTTGTGGG CGACTGCGCG GGACTGCCCG 4020
   AACAGACGAG GGCCGTAGGC GAATGTCTGT TCGACACTGG CAGAGGCCCT CGACGTACAC 4080
   AGTCTCCAAA AGTGGCAGTA GTGGCTTTGC GCGCTACTGC TTTCCCGGAG CACTATGCGG 4140
   ATAAAAATAT CCAATTACAG TACTATTATT ACCAAAGAAT CTGCAGTCCA CCGTGAAAAG 4200
   CCCCTTTACA CGCGCCTTGG GGATAAACAA ATAAAAAGAT TTATGTAAGT TTATACATAG 4260
   GCGAGTACTC TGTTATTGGG ACTATTTACG AAGTTATTAT AACTTTTTCC TTCTCATACT 4320
   CATAAGTTGT AAAGGCACAG CGGGAATAAG GGAAAAAACG CCGTAAAACG GAAGGACAAA 4380
   AACGAGTGGG TCTTTGCGAC CACTTTCATT TTCTACGACT TCTAGTCAAC CCACGTGCTC 4440
   ACCCAATGTA GCTTGACCTA GAGTTGTCGC CATTCTAGGA ACTCTCAAAA GCGGGGCTTC 4500
   TTGCAAAAGG TTACTACTCG TGAAAATTTC AAGACGATAC ACCGCGCCAT AATAGGGCAT 4560
   AACTGCGGCC CGTTCTCGTT GAGCCAGCGG CGTATGTGAT AAGAGTCTTA CTGAACCAAC 4620
   TCATGAGTGG TCAGTGTCTT TTCGTAGAAT GCCTACCGTA CTGTCATTCT CTTAATACGT 4680
   CACGACGGTA TTGGTACTCA CTATTGTGAC GCCGGTTGAA TGAAGACTGT TGCTAGCCTC 4740
   CTGGCTTCCT CGATTGGCGA AAAAACGTGT TGTACCCCCT AGTACATTGA GCGGAACTAG 4800
   CAACCCTTGG CCTCGACTTA CTTCGGTATG GTTTGCTGCT CGCACTGTGG TGCTACGGAC 4860
   ATCGTTACCG TTGTTGCAAC GCGTTTGATA ATTGACCGCT TGATGAATGA GATCGAAGGG 4920
   CCGTTGTTAA TTATCTGACC TACCTCCGCC TATTTCAACG TCCTGGTGAA GACGCGAGCC 4980
   GGGAAGGCCG ACCGACCAAA TAACGACTAT TTAGACCTCG GCCACTCGCA CCCAGAGCGC 5040
   CATAGTAACG TCGTGACCCC GGTCTACCAT TCGGGAGGGC ATAGCATCAA TAGATGTGCT 5100
   GCCCCTCAGT CCGTTGATAC CTACTTGCTT TATCTGTCTA GCGACTCTAT CCACGGAGTG 5160
   ACTAATTCGT AACCATTGAC AGTCTGGTTC AAATGAGTAT ATATGAAATC TAACTAAATT 5220
   TTGAAGTAAA AATTAAATTT TCCTAGATCC ACTTCTAGGA AAAACTATTA GAGTACTGGT 5280
   TTTAGGGAAT TGCACTCAAA AGCAAGGTGA CTCGCAGTCT GGGGCATCTT TTCTAGTTTC 5340
   CTAGAAGAAC TCTAGGAAAA AAAGACGCGC ATTAGACGAC GAACGTTTGT TTTTTTGGTG 5400
   GCGATGGTCG CCACCAAACA AACGGCCTAG TTCTCGATGG TTGAGAAAAA GGCTTCCATT 5460
   GACCGAAGTC GTCTCGCGTC TATGGTTTAT GACAGGAAGA TCACATCGGC ATCAATCCGG 5520
   TGGTGAAGTT CTTGAGACAT CGTGGCGGAT GTATGGAGCG AGACGATTAG GACAATGGTC 5580
   ACCGACGACG GTCACCGCTA TTCAGCACAG AATGGCCCAA CCTGAGTTCT GCTATCAATG 5640
   GCCTATTCCG CGTCGCCAGC CCGACTTGCC CCCCAAGCAC GTGTGTCGGG TCGAACCTCG 5700
   CTTGCTGGAT GTGGCTTGAC TCTATGGATG TCGCACTCGT AACTCTTTCG CGGTGCGAAG 5760
   GGCTTCCCTC TTTCCGCCTG TCCATAGGCC ATTCGCCGTC CCAGCCTTGT CCTCTCGCGT 5820
   GCTCCCTCGA AGGTCCCCCT TTGCGGACCA TAGAAATATC AGGACAGCCC AAAGCGGTGG 5880
   AGACTGAACT CGCAGCTAAA AACACTACGA GCAGTCCCCC CGCCTCGGAT ACCTTTTTGC 5940
   GGTCGTTGCG CCGGAAAAAT GCCAAGGACC GGAAAACGAC CGGAAAACGA GTGTACAAGA 6000
   AAGGACGCAA TAGGGGACTA AGACACCTAT TGGCATAATG GCGGAAACTC ACTCGACTAT 6060
   GGCGAGCGGC GTCGGCTTGC TGGCTCGCGT CGCTCAGTCA CTCGCTCCTT CGCCTTCTCG 6120
   CGGGTTATGC GTTTGGCGGA GAGGGGCGCG CAACCGGCTA AGTAATTACG TCGACCGTGC 6180
   TGTCCAAAGG GCTGACCTTT CGCCCGTCAC TCGCGTTGCG TTAATTACAC TCAATCGAGT 6240
   GAGTAATCCG TGGGGTCCGA AATGTGAAAT ACGAAGGCCG AGCATACAAC ACACCTTAAC 6300
   ACTCGCCTAT TGTTAAAGTG TGTCCTTTGT CGATACTGGT ACTAATGCGG 6350
(2) INFORMATION FOR SEQ ID NO:10:
   (i) SEQUENCE CHARACTERISTICS:
      (A) LENGTH: 713 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:10:
   AAGCTTGGGC TGCAGGTCGA TCGACTCTAG AGGATCGATC CCCACCATGG GTCAATCACG 60
   CTACCTCCTC TTTTTGGCCA CCCTTGCCCT CCTAAACCAC CTCAGTTTGG CCAGGGTCAT 120
   TCCAGTCTCT GGACCTGCCA GGTGTCTTAG CCAGTCCCGA AACCTGCTGA AGACCACAGA 180
   TGACATGGTG AAGACGGCCA GAGAAAAACT GAAACATTAT TCCTGCACTG CTGAAGACAT 240
   CGATCATGAA GACATCACAC GGGACCAAAC CAGCACATTG AAGACCTGTT TACCACTGGA 300
   ACTACACAAG AACGAGAGTT GCCTGGCTAC TAGAGAGACT TCTTCCACAA CAAGAGGGAG 360
   CTGCCTGCCC CCACAGAAGA CGTCTTTGAT GATGACCCTG TGCCTTGGTA GCATCTATGA 420
   GGACTTGAAG ATGTACCAGA CAGAGTTCCA GGCCATCAAC GCAGCACTTC AGAATCACAA 480
   CCATCAGCAG ATCATTCTAG ACAAGGGCAT GCTGGTGGCC ATCGATGAGC TGATGCAGTC 540
   TCTGAATCAT AATGGCGAGA CTCTGCGCCA GAAACCTCCT GTGGGAGAAG CAGACCCTTA 600
   CAGAGTGAAA ATGAAGCTCT GCATCCTGCT TCACGCCTTC AGCACCCGCG TCGTGACCAT 660
   CAACAGGGTG ATGGGCTATC TGAGCTCCGC CTGAGAATTC ATTGATCCAC TAG 713
(2) INFORMATION FOR SEQ ID NO:11:
   (i) SEQUENCE CHARACTERISTICS:
      (A) LENGTH: 713 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:11:
   TTCGAACCCG ACGTCCAGCT AGCTGAGATC TCCTAGCTAG GGGTGGTACC CAGTTAGTGC 60
   GATGGAGGAG AAAAACCGGT GGGAACGGGA GGATTTGGTG GAGTCAAACC GGTCCCAGTA 120
   AGGTCAGAGA CCTGGACGGT CCACAGAATC GGTCAGGGCT TTGGACGACT TCTGGTGTCT 180
   ACTGTACCAC TTCTGCCGGT CTCTTTTTGA CTTTGTAATA AGGACGTGAC GACTTCTGTA 240
   GCTAGTACTT CTGTAGTGTG CCCTGGTTTG GTCGTGTAAC TTCTGGACAA ATGGTGACCT 300
   TGATGTGTTC TTGCTCTCAA CGGACCGATG ATCTCTCTGA AGAAGGTGTT GTTCTCCCTC 360
   GACGGACGGG GGTGTCTTCT GCAGAAACTA CTACTGGGAC ACGGAACCAT CGTAGATACT 420
   CCTGAACTTC TACATGGTCT GTCTCAAGGT CCGGTAGTTG CGTCGTGAAG TCTTAGTGTT 480
   GGTAGTCGTC TAGTAAGATC TGTTCCCGTA CGACCACCGG TAGCTACTCG ACTACGTCAG 540
   AGACTTAGTA TTACCGCTCT GAGACGCGGT CTTTGGAGGA CACCCTCTTC GTCTGGGAAT 600
   GTCTCACTTT TACTTCGAGA CGTAGGACGA AGTGCGGAAG TCGTGGGCGC AGCACTGGTA 660
   GTTGTCCCAC TACCCGATAG ACTCGAGGCG GACTCTTAAG TAACTAGGTG ATC 713
(2) INFORMATION FOR SEQ ID NO:12:
   (i) SEQUENCE CHARACTERISTICS:
      (A) LENGTH: 215 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:12:
   Met Gly Gln Ser Arg Tyr Leu Leu Phe Leu Ala Thr Leu Ala Leu Leu
   1 5 10 15
   Asn His Leu Ser Leu Ala Arg Val Ile Pro Val Ser Gly Pro Ala Arg
   20 25 30
   Cys Leu Ser Gln Ser Arg Asn Leu Leu Lys Thr Thr Asp Asp Met Val
   35 40 45
   Lys Thr Ala Arg Glu Lys Leu Lys His Tyr Ser Cys Thr Ala Glu Asp
   50 55 60
   Ile Asp His Glu Asp Ile Thr Arg Asp Gln Thr Ser Thr Leu Lys Thr
   65 70 75 80
   Cys Leu Pro Leu Glu Leu His Lys Asn Glu Ser Cys Leu Ala Thr Arg
   85 90 95
   Glu Thr Ser Ser Thr Thr Arg Gly Ser Cys Leu Pro Pro Gln Lys Thr
   100 105 110
   Ser Leu Met Met Thr Leu Cys Leu Gly Ser Ile Tyr Glu Asp Leu Lys
   115 120 125
   Met Tyr Gln Thr Glu Phe Gln Ala Ile Asn Ala Ala Leu Gln Asn His
   130 135 140
   Asn His Gln Gln Ile Ile Leu Asp Lys Gly Met Leu Val Ala Ile Asp
   145 150 155 160
   Glu Leu Met Gln Ser Leu Asn His Asn Gly Glu Thr Leu Arg Gln Lys
   165 170 175
   Pro Pro Val Gly Glu Ala Asp Pro Tyr Arg Val Lys Met Lys Leu Cys
   180 185 190
   Ile Leu Leu His Ala Phe Ser Thr Arg Val Val Thr Ile Asn Arg Val
   195 200 205
   Met Gly Tyr Leu Ser Ser Ala
   210 215
(2) INFORMATION FOR SEQ ID NO:13:
   (i) SEQUENCE CHARACTERISTICS:
      (A) LENGTH: 1060 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:13:
   AAGCTTGGGC TGCAGGTCGA TCGACTCTAG AGGATCGATC CCCACCATGG GTCCTCAGAA 60
   GCTAACCATC TCCTGGTTTG CCATCGTTTT GCTGGTGTCT CCACTCATGG CCATGTGGGA 120
   GCTGAGAAAG ACGTTTATGT TGTAGAGGTG GACTGGACTC CCGATGCCCC TGGAGAAACA 180
   GTGAACCTCA CCTGTGACAC GCCTGAAGAA GATGACATCA CCTGGACCTC AGACCAGAGA 240
   CATGGAGTCA TAGGCTCTGG AAAGACCCTG ACCATCACTG TCAAAGAGTT TCTAGATGCT     300
   GGCCAGTACA CCTGCCACAA AGGAGGCGAG ACTCTGAGCC ACTCACATCT GCTGCTCCAC     360
   AAGAAGGAAA ATGGAATTTG GTCCACTGAA ATTTTAAAAA ATTTCAAAAA CAAGACTTTC     420
   CTGAAGTGTG AAGCACCAAA TTACTCCGGA CGGTTCACGT GCTCATGGCT GGTGCAAAGA     480
   AACATGGACT TGAAGTTCAA CATCAAGAGC AGTAGCAGTT CCCCTGACTC TCGGGCAGTG     540
   ACATGTGGAA TGGCGTCTCT GTCTGCAGAG AAGGTCACAC TGGACCAAAG GGACTATGAG     600
   AAGTATTCAG TGTCCTGCCA GGAGGATGTC ACCTGCCCAA CTGCCGAGGA GACCCTGCCC     660
   ATTGAACTGG CGTTGGAAGC ACGGCAGCAG AATAAATATG AGAACTACAG CACCAGCTTC     720
   TTCATCAGGG ACATCATCAA ACCAGACCCG CCCAAGAACT TGCAGATGAA GCCTTTGAAG     780
   AACTCACAGG TGGAGGTCAG CTGGGAGTAC CCTGACTCCT GGAGCACTCC CCATTCCTAC     840
   TTCTCCCTCA AGTTCTTTGT TCGAATCCAG CGCAAGAAAG AAAAGATGAA GGAGACAGAG     900
   GAGGGGTGTA ACCAGAAAGG TGCGTTCCTC GTAGAGAAGA CATCTACCGA AGTCCAATGC     960
   AAAGGCGGGA ATGTCTGCGT GCAAGCTCAG GATCGCTATT ACAATTCCTC ATGCAGCAAG    1020
   TGGGCATGTG TTCCCTGCAG GGTCCGATCC TAGGAATTCC                         1060
(2) INFORMATION FOR SEQ ID NO:14:
   (i) SEQUENCE CHARACTERISTICS:
      (A) LENGTH: 1060 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:14:
   TTCGAACCCG ACGTCCAGCT AGCTGAGATC TCCTAGCTAG GGGTGGTACC CAGGAGTCTT 60
   CGATTGGTAG AGGACCAAAC GGTAGCAAAA CGACCACAGA GGTGAGTACC GGTACACCCT 120
   CGACCTCTTT CTGCAAATAC AACATCTCCA CCTGACCTGA GGGCTACGGG GACCTCTTTG 180
   TCACTTGGAG TGGACACTGT GCGGACTTCT TCTACTGTAG TGGACCTGGA GTCTGGTCTC 240
   TGTACCTCAG TATCCGAGAC CTTTCTGGGA CTGGTAGTGA CAGTTTCTCA AAGATCTACG 300
   ACCGGTCATG TGGACGGTGT TTCCTCCGCT CTGAGACTCG GTGAGTGTAG ACGACGAGGT 360
   GTTCTTCCTT TTACCTTAAA CCAGGTGACT TTAAAATTTT TTAAAGTTTT TGTTCTGAAA 420
   GGACTTCACA CTTCGTGGTT TAATGAGGCC TGCCAAGTGC ACGAGTACCG ACCACGTTTC 480
   TTTGTACCTG AACTTCAAGT TGTAGTTCTC GTCATCGTCA AGGGGACTGA GAGCCCGTCA 540
   CTGTACACCT TACCGCAGAG ACAGACGTCT CTTCCAGTGT GACCTGGTTT CCCTGATACT 600
   CTTCATAAGT CACAGGACGG TCCTCCTACA GTGGACGGGT TGACGGCTCC TCTGGGACGG 660
   GTAACTTGAC CGCAACCTTC GTGCCGTCGT CTTATTTATA CTCTTGATGT CGTGGTCGAA 720
   GAAGTAGTCC CTGTAGTAGT TTGGTCTGGG CGGGTTCTTG AACGTCTACT TCGGAAACTT 780
   CTTGAGTGTC CACCTCCAGT CGACCCTCAT GGGACTGAGG ACCTCGTGAG GGGTAAGGAT 840
   GAAGAGGGAG TTCAAGAAAC AAGCTTAGGT CGCGTTCTTT CTTTTCTACT TCCTCTGTCT 900
   CCTCCCCACA TTGGTCTTTC CACGCAAGGA GCATCTCTTC TGTAGATGGC TTCAGGTTAC 960
   GTTTCCGCCC TTACAGACGC ACGTTCGAGT CCTAGCGATA ATGTTAAGGA GTACGTCGTT 1020
   CACCCGTACA CAAGGGACGT CCCAGGCTAG GATCTTAAGG 1060
(2) INFORMATION FOR SEQ ID NO:15:
   (i) SEQUENCE CHARACTERISTICS:
      (A) LENGTH: 335 amino acids
      (B) TYPE: amino acid
      (C) STRANDEDNESS: unknown
      (D) TOPOLOGY: unknown
   (ii) MOLECULE TYPE: protein
   (xi) SEQUENCE DESCRIPTION: SEQ ID NO:15:
   Met Gly Pro Gln Lys Leu Thr Ile Ser Trp Phe Ala Ile Val Leu Leu
   1 5 10 15
   Val Ser Pro Leu Met Ala Met Trp Glu Leu Glu Lys Asp Val Tyr Val
   20 25 30
   Val Glu Val Asp Trp Thr Pro Asp Ala Pro Gly Glu Thr Val Asn Leu
   35 40 45
   Thr Cys Asp Thr Pro Glu Glu Asp Asp Ile Thr Trp Thr Ser Asp Gln
   50 55 60
   Arg His Gly Val Ile Gly Ser Gly Lys Thr Leu Thr Ile Thr Val Lys
   65 70 75 80
   Glu Phe Leu Asp Ala Gly Gln Tyr Thr Cys His Lys Gly Gly Glu Thr
   85 90 95
   Leu Ser His Ser His Leu Leu Leu His Lys Lys Glu Asn Gly Ile Trp
   100 105 110
   Ser Thr Glu Ile Leu Lys Asn Phe Lys Asn Lys Thr Phe Leu Lys Cys
   115 120 125
   Glu Ala Pro Asn Tyr Ser Gly Arg Phe Thr Cys Ser Trp Leu Val Gln
   130 135 140
   Arg Asn Met Asp Leu Lys Phe Asn Ile Lys Ser Ser Ser Ser Ser Pro
   145 150 155 160
   Asp Ser Arg Ala Val Thr Cys Gly Met Ala Ser Leu Ser Ala Glu Lys
   165 170 175
   Val Thr Leu Asp Gln Arg Asp Tyr Glu Lys Tyr Ser Val Ser Cys Gln
               180                 185                 190
   Glu Asp Val Thr Cys Pro Thr Ala Glu Glu Thr Leu Pro Ile Glu Leu
           195                 200                 205
   Ala Leu Glu Ala Arg Gln Gln Asn Lys Tyr Glu Asn Tyr Ser Thr Ser
       210                 215                 220
   Phe Phe Ile Arg Asp Ile Ile Lys Pro Asp Pro Pro Lys Asn Leu Gln
   225                 230                 235                 240
   Met Lys Pro Leu Lys Asn Ser Gln Val Glu Val Ser Trp Glu Tyr Pro
                   245                 250                 255
   Asp Ser Trp Ser Thr Pro His Ser Tyr Phe Ser Leu Lys Phe Phe Val
               260                 265                 270
   Arg Ile Gln Arg Lys Lys Glu Lys Met Lys Glu Thr Glu Glu Gly Cys
           275                 280                 285
   Asn Gln Lys Gly Ala Phe Leu Val Glu Lys Thr Ser Thr Glu Val Gln
       290                 295                 300
   Cys Lys Gly Gly Asn Val Cys Val Gln Ala Gln Asp Arg Tyr Tyr Asn
   305                 310                 315                 320
   Ser Ser Cys Ser Lys Trp Ala Cys Val Pro Cys Arg Val Arg Ser
                   325                 330                 335
(2) INFORMATION FOR SEQ ID NO:16:
   (i) SEQUENCE CHARACTERISTICS:
      (A) LENGTH: 61 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:16:
   CCGCCGGTGG CGGTGGCTCG GGCGGTGGTG GGTCGGGTGG CGGCGGATCT TCCATGGAGC 60
   T 61
(2) INFORMATION FOR SEQ ID NO:17:
   (i) SEQUENCE CHARACTERISTICS:
      (A) LENGTH: 61 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:17:
   CCATGGAAGA TCCGCCGCCA CCCGACCCAC CACCGCCCGA GCCACCGCCA CCGGCGGAGC 60
   T 61
(2) INFORMATION FOR SEQ ID NO:18:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:18:
      CAGAGTGAAA ATGAAGCT 18
(2) INFORMATION FOR SEQ ID NO:19:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:19:
      GAAGCTCTGC ATCCTGCT 18
(2) INFORMATION FOR SEQ ID NO:20:
   (i) SEQUENCE CHARACTERISTICS:
      (A) LENGTH: 62 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:20:
   GGGTCCGATC CGGTGGCGGT GGCTCGGGCG GTGGTGGGTC GGGTGGCGGC GGATCTTCCA 60
   TG 62
(2) INFORMATION FOR SEQ ID NO:21:
   (i) SEQUENCE CHARACTERISTICS:
      (A) LENGTH: 70 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:21:
   GATCCATGGA AGATCCGCCG CCACCCGACC CACCACCGCC CGAGCCACCG CCACCGGATC 60
   GGACCCTGCA 70
(2) INFORMATION FOR SEQ ID NO:22:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:22:
      CTATTACAAT TCCTCATG 18
(2) INFORMATION FOR SEQ ID NO:23:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:23:
      GAGGGCAAGG GTGGCCAA 18
(2) INFORMATION FOR SEQ ID NO:24:
   (i) SEQUENCE CHARACTERISTICS:
      (A) LENGTH: 67 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:24:
   TTGCTGGAGC TCCGCCGGTG GCGGTGGCTC GGGCGGTGGT GGGTCGGGTG GCGGCGGATC 60
   TATGTGG 67
(2) INFORMATION FOR SEQ ID NO:25:
   (i) SEQUENCE CHARACTERISTICS:
      (A) LENGTH: 67 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:25:
   CACATAGATC CGCCGCCACC CGACCCACCA CCGCCCGAGC CACCGCCACC GGCGGAGCTC 60
   CAGCAAA 67
(2) INFORMATION FOR SEQ ID NO:26:
   (i) SEQUENCE CHARACTERISTICS:
      (A) LENGTH: 82 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:26:
   CTGGCCTGCA GGGTCCGATC CGGTGGCGGT GGCTCGGGCG GTGGTGGGTC GGGTGGCGGC 60
   GGATCTAGGG TCATTCCAGT CT 82
(2) INFORMATION FOR SEQ ID NO:27:
   (i) SEQUENCE CHARACTERISTICS:
      (A) LENGTH: 82 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:27:
   CTGGAATGAC CCTAGATCCG CCGCCACCCG ACCCACCACC GCCCGAGCCA CCGCCACCGG 60
   ATCGGACCCT GCAGGCCAGA GA 82
(2) INFORMATION FOR SEQ ID NO:28:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:28:
      GCAAAGGCGG GAATGTCT 18
(2) INFORMATION FOR SEQ ID NO:29:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:29:
      AGGAATAATG TTTCAGTT 18
(2) INFORMATION FOR SEQ ID NO:30:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:30:
      CAGCAGTGCA GGAATAAT 18
(2) INFORMATION FOR SEQ ID NO:31:
   (i) SEQUENCE CHARACTERISTICS:
      (A) LENGTH: 75 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:31:
   CCCTGCAGGG TCCGATCCGG TGGCGGTGGC TCGGGCGGTG GTGGGTCGGG TGGCGGCGGA 60
   TCTTCCATGG GTCAA 75
(2) INFORMATION FOR SEQ ID NO:32:
   (i) SEQUENCE CHARACTERISTICS:
      (A) LENGTH: 75 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:32:
   CCCTGCAGGG TCCGATCCGG TGGCGGTGGC TCGGGCGGTG GTGGGTCGGG TGGCGGCGGA 60
   TCTTCCATGG GTCAA 75
(2) INFORMATION FOR SEQ ID NO:33:
   (i) SEQUENCE CHARACTERISTICS:
      (A) LENGTH: 96 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:33:
   TGTGTTCCCT GCAGGGTCCG ATCCGGTGGC GGTGGCTCGG GCGGTGGTGG GTCGGGTGGC 60
   GGCGGATCTA GGGTCATTCC AGTCTCTGGA CCTGCC 96
(2) INFORMATION FOR SEQ ID NO:34:
   (i) SEQUENCE CHARACTERISTICS:
      (A) LENGTH: 18 base pairs
      (B) TYPE: nucleic acid
      (C) STRANDEDNESS: unknown
      (D) TOPOLOGY: unknown
   (ii) MOLECULE TYPE: DNA (genomic)
   (xi) SEQUENCE DESCRIPTION: SEQ ID NO:34:
      GTCATCTTCT TCAGGCGT 18
(2) INFORMATION FOR SEQ ID NO:35:
   (i) SEQUENCE CHARACTERISTICS:
      (A) LENGTH: 33 base pairs
      (B) TYPE: nucleic acid
      (C) STRANDEDNESS: single
      (D) TOPOLOGY: linear
   (ii) MOLECULE TYPE: DNA (genomic)
   (xi) SEQUENCE DESCRIPTION: SEQ ID NO:35:
      TGCAGTGGTG GCGGTGGCGG CGGATCTAGA AAC 33
(2) INFORMATION FOR SEQ ID NO:36:
   (i) SEQUENCE CHARACTERISTICS:
      (A) LENGTH: 7 amino acids
      (B) TYPE: amino acid
      (C) STRANDEDNESS:
      (D) TOPOLOGY: linear
   (ii) MOLECULE TYPE: peptide
   (xi) SEQUENCE DESCRIPTION: SEQ ID NO:36:
   Gly Gly Gly Gly Gly Gly Ser
   1               5